# EUROPEAN PATENT APPLICATION

(11) **EP 3 118 808 A1**
(43) Date of publication of application: **18.01.2017**
(21) Application number: 15761444.7
(22) Date of filing: 10.03.2015
(51) Int. Cl.: G06Q 50/22

(54) **TEAM MEDICAL SUPPORT DEVICE, CONTROL METHOD FOR TEAM MEDICAL SUPPORT DEVICE, TEAM MEDICAL SUPPORT PROGRAM, AND TEAM MEDICAL SUPPORT SYSTEM**

(30) Priority: 13.03.2014 JP 2014050345
(71) Applicant: Fujifilm Corporation, Minato-ku Tokyo 106-8620 (JP)
(72) Inventor: KUDOU,Yuuya, Tokyo 107-0052 (JP); UEDA, Satoshi, Tokyo 107-0052 (JP); MATSUMASA, Hironori, Tokyo 107-0052 (JP); OHTA, Yasunori, Tokyo 107-0052 (JP)
(74) Representative: Klunker . Schmitt-Nilson . Hirsch
(86) International application number: PCT/JP2015/057037
(87) International publication number: WO 2015/137349

(57) **Abstract**

Provided are a team medical support device, a method for controlling a team medical support device, a team medical support device control program, and a team medical support system which can smoothly perform information exchange and information sharing in a team medical treatment.

A team medical support device (11) creates and transmits a first medical support screen (20) or a second medical support screen (21) on the basis of patient schedule information, staff schedule information, and communication messages in response to a transmission request from client terminals (13a) to (13c). A patient schedule display region (20a) and a patient-related message display region (20b) are displayed in parallel on the first medical support screen (20). A staff schedule display region (21a) and a staff-related message display region (21b) are displayed in parallel on the second medical support screen (21).

## Description

### Field of the Invention

The present invention relates to a team medical support device, a method for controlling a team medical support device, a team medical support program, and a team medical support system which support a team medical treatment in which a plurality of medical staff members examine a patient in cooperation with each other.

In the medical field, a team medical treatment in which a medical team including medical staff members, such as doctors or nurses, examines a patient is performed. In addition, the use of a system in which patient schedule information (which is also referred to as a clinical path) indicating a plan for examining a patient is introduced into a team medical treatment and a medical team examines a patient on the basis of the patient schedule information has increased. The plan for examining a patient is a plan for a medical procedure that is scheduled to be performed for a patient, such as an examination, a cure, or a medical treatment. Patient event information indicating a medical procedure, such as an examination, a cure, or a medical treatment, is recorded in the patient schedule information in chronological order. In the team medical treatment, it is important to smoothly exchange and share information between a plurality of medical staff members and thus to strengthen the cooperation between the medical staff members. As a device that can be used for smooth information exchange and sharing between medical staff members, JP2011-238096A (US2012/112702A) discloses a schedule management system and JP2007-249251A (US2012/112702A) discloses a clinical communication device.

In the schedule management system disclosed in JP2011-238096A (US2012/112702A), patient schedule information and staff schedule information, which is a medical staff work schedule, are managed in a database. The staff schedule information is schedule information indicating the relationship between the patient and work that the medical staff is in charge of. Staff event information indicating the work performed by the medical staff is recorded in the staff schedule information in chronological order.

In the schedule management system, the client terminal of the medical staff can access a server that manages the patient schedule information and the staff schedule information. At the time that the client terminal accesses the patient schedule information, the medical staff member designates one patient of which the information is desired to be transmitted and transmits a transmission request including designation information to the server. At the time that the client terminal accesses the staff schedule information, a medical staff member designates his or her staff ID and transmits a transmission request including designation information to the server. For the staff ID, for example, in a case in which the client terminal is allocated to each medical staff member and the terminal ID of the client terminal corresponds to the staff ID, the terminal ID is given as staff ID designation information to the transmission request. The server selectively transmits the patient schedule information of one patient or the staff schedule information of one medical staff member in response to a request from the client terminal. Specifically, the server selectively transmits two types of screens, that is, a screen on which the patient schedule information is displayed and a screen on which the staff schedule information is displayed.

According to the schedule management system, a medical staff member can check, for example, the patient schedule information of the patient that the medical staff member is in charge of, in addition to his or her staff schedule information, through the client terminal. In a team medical treatment, since a plurality of medical staff members are related to one patient, each medical staff member can check the patient schedule information. Therefore, the medical staff members smoothly exchange and share information and it is possible to strengthen the cooperation between a plurality of medical staff members.

In the clinical communication device disclosed in JP2007-249251A (US2012/112702A), similarly to the schedule management system disclosed in JP2011-238096A (US2012/112702A), patient schedule information and staff schedule information are managed in a database. Similarly to the schedule management system disclosed in JP2011-238096A (US2012/112702A), the clinical communication device disclosed in JP2007-249251A (US2012/112702A) can be accessed by the client terminal of the medical staff and transmits the patient schedule information of the patient and the staff schedule information of the medical staff in response to a request from the client terminal.

In JP2007-249251A (US2012/112702A), unlike JP2011-238096A (US2012/112702A), a communicator screen in which the patient schedule information and the staff schedule information are mixed on one screen is created and transmitted. On the communicator screen, a staff schedule display region in which the staff schedule information of a designated medical staff member is displayed and a patient schedule display region in which the patient schedule information of a plurality of patients that the designated medical staff member is in charge of is displayed are provided in parallel in one screen. The schedules of a plurality of patients are displayed side by side in the patient schedule display region.

In the communicator screen disclosed in JP2007-249251A (US2012/112702A), in a case in which there is a communication message related to a certain patient, such as an item to be transmitted between medical staff members, a message mark indicating whether a communication message is present is displayed in the patient schedule display region so as to be associated with the patient schedule information. At the time that the message mark is clicked, the text of the communication message is displayed. The client terminal displays the communicator screen transmitted from the clinical communication device. Therefore, the medical staff members smoothly exchange and share information and it is possible to strengthen the cooperation between a plurality of medical staff members.

### SUMMARY OF THE INVENTION

### Problems to Be Solved by The Invention

However, the techniques disclosed in JP2011-238096A (US2012/112702A) and JP2007-249251A (US2012/112702A) are not enough to achieve smooth information exchange and information sharing in the team medical treatment. First, in JP2011-238096A (US2012/112702A), since a message display function for displaying the communication messages exchanged between the medical staff members is not provided, it is difficult to rapidly check the communication messages. It is indispensable to check the communication messages between the medical staff members in the team medical treatment in order to strengthen the cooperation between the medical staff members. The schedule management system disclosed in JP2011-238096A (US2012/112702A) needs to be improved since it does not have the message display function.

Second, in the clinical communication device disclosed in JP2007-249251A (US2012/112702A), a plurality of patient schedule information items and staff schedule information items are displayed in parallel on one screen and a message mark is also displayed in the patient schedule information display region. Therefore, the type or number of information items is too large and the information is difficult to see.

In addition, the communicator screen disclosed in JP2007-249251A (US2012/112702A) is provided with the message display function, but it is difficult to ascertain the relation between the communication message and the staff schedule information. Specifically, in the communicator screen disclosed in JP2007-249251A (US2012/112702A), since the message mark is displayed in the patient schedule information display region, it is possible to ascertain the relation between the communication message and the patient. However, in the team medical treatment, since a plurality of staff members are related to one patient, a plurality of communication messages related to the patient is likely to include communication messages that are not related to a medical staff member who has requested the communicator screen. In this case, simply displaying the communication message in each patient schedule information display region is not enough to allow the relation between one medical staff member who has requested the communicator screen and the communication message to be ascertained. In the team medical treatment, it is important to ascertain the communication messages exchanged for one patient and to rapidly ascertain the content of the communication messages related to each medical staff member with high accuracy.

In the communicator screen disclosed in JP2007-249251A (US2012/112702A), it is difficult to collectively check the communication messages related to the medical procedure that is scheduled to be performed for a patient or the work that is scheduled to be performed by the medical staff. In the team medical treatment, in many cases, the communication messages related to the entire schedule of a patient or the medical staff, such as a plan for examining a patient or the work schedule of the medical staff, are exchanged and the communication messages related to a medical procedure for a patient, such as an examination, a cure, or a medical treatment, or the work of the medical staff are exchanged. Therefore, there is a demand for collectively checking the communication messages related to a medical procedure or work in order to rapidly and accurately ascertain the content of a medical procedure for a patient or the work of the medical staff. However, in the communicator screen disclosed in JP2007-249251A (US2012/112702A), it is necessary to search for a communication message related to a specific medical procedure or work from a plurality of communication messages and to check the communication message. As a result, the check process is very complicated and the medical staff is likely to miss a communication message.

As such, in the clinical communication device disclosed in JP2007-249251A (US2012/112702A), the following problems need to be solved: since the type or number of information items displayed on one screen is large, the information is difficult to see; it is difficult to rapidly ascertain the relation between the communication messages and the medical staff; and it is difficult to collectively check the communication messages related to a medical procedure for a patient or the work of the medical staff.

An object of the invention is to provide a team medical support device, a method for controlling a team medical support device, a team medical support device control program, and a team medical support system which can smoothly perform information exchange and information sharing in a team medical treatment.

### Means for Solving the Problems

In order to solve the above-mentioned problems, a team medical support device according to the invention comprises a patient schedule information storage unit, a staff schedule information storage unit, a communication message storage unit, a receiving unit, a screen creation unit, and a transmission unit. The patient schedule information storage unit stores patient schedule information of a plurality of patients in which patient event information indicating a medical procedure that is scheduled to be performed for the patient is registered. The staff schedule information storage unit stores staff schedule information of a plurality of staff members in which staff event information indicating the work that is scheduled to be performed by the medical staff member is registered. The communication message storage unit stores a plurality of communication messages which are exchanged between client terminals used by the plurality of medical staff members. The receiving unit receives a medical support screen transmission request including designation information for designating one patient or one medical staff member among the plurality of patients or the plurality of medical staff members. The screen creation unit creates two types of medical support screens, that is, a first medical support screen and a second medical support screen, on the basis of the patient schedule information, the staff schedule information, and the communication messages. A patient schedule display region in which the patient schedule information of the patient designated by the designation information is displayed and a patient-related message display region in which a communication message related to the patient designated by the designation information or the patient event information of the patient is displayed are displayed in parallel on the first medical support screen. A staff schedule display region in which the staff schedule information of the medical staff member designated by the designation information is displayed and a staff-related message display region in which a communication message related to the medical staff member designated by the designation information or the staff event information of the medical staff member is displayed are displayed in parallel on the second medical support screen. The transmission unit transmits the first medical support screen or the second medical support screen which is created on the basis of the transmission request to the client terminals.

It is preferable that a transmission date and time of the communication message is displayed in the patient-related message display region and the staff-related message display region. It is preferable that a transmission source and a transmission destination of the communication message are displayed in at least one of the patient-related message display region or the staff-related message display region. A title of the communication message or at least a portion of the text of the communication message may be displayed in at least one of the patient-related message display region or the staff-related message display region.

The screen creation unit may display the communication message in the patient-related message display region in an order of a transmission date and time and set a display order of the communication messages in the staff-related message display region according to a degree of urgency of the communication message.

The team medical support device may further comprise an access authority information storage unit that stores access authority information of the medical staff member for the patient-related message display region. In this case, in a condition of creating the first medical support screen, the screen creation unit may check the access authority information of the medical staff member, who is a request source requesting the transmission of the first medical support screen. In a case in which the communication messages displayed in the patient-related message display region include a communication message that is not allowed to be accessed by the medical staff member, it is preferable that the screen creation unit performs a concealment process of concealing the communication message that is not allowed to be accessed. In the concealment process, information indicating that the communication message that is not allowed to be accessed by the medical staff member is present may be displayed.

The team medical support device may further comprise a message relay unit that receives the communication message transmitted from the client terminal and relays the communication message and a reception notification unit that transmits a notification indicating the reception of the communication message to the client terminal used by the medical staff member who is a transmission destination of the received communication message.

The team medical support device may further include a voice recognition unit that, in a case in which audio data is received as the communication message from the client terminal, converts the audio data into text data and stores the text data in the communication message storage unit. In this case, it is preferable that the voice recognition unit converts medical terms in the audio data into text data on the basis of a medical term dictionary that stores the medical terms.

The team medical support device may further include a message analysis unit that analyzes the content of the communication message received from the client terminal and an order processing unit that, in a case in which the content of the communication message instructs a treatment or an examination for a patient, orders the treatment or the examination on the basis of the content of the communication message.

A method for controlling a team medical support device according to the invention comprises a receiving step, an information reading step, a screen creation step, and a transmission step. In the information reading step, a medical support screen transmission request including designation information for designating one patient or one medical staff member among a plurality of patients or a plurality of medical staff members is received. In the information reading step, a patient schedule information storage unit, a staff schedule information storage unit, and a communication message storage unit are accessed and patient schedule information, staff schedule information, and communication messages are read. In the screen creation step, two types of medical support screens, that is, a first medical support screen and a second medical support screen are created, on the basis of the patient schedule information, the staff schedule information, and the communication messages. In the transmission step, the first medical support screen or the second medical support screen which is created on the basis of the transmission request is transmitted to client terminals.

A team medical support program according to the invention causes a computer to perform a receiving step, an information reading step, a screen creation step, and a transmission step.

A team medical support system according to the invention comprises client terminals that are used by a plurality of medical staff members and a team medical support device that creates a medical support screen on the basis of a request from the client terminals and transmits the medical support screen to the client terminals. The team medical support device comprises a patient schedule information storage unit, a staff schedule information storage unit, a communication message storage unit, a receiving unit, a screen creation unit, and a transmission unit.

The team medical support device according to the invention creates the first medical support screen of the patient designated by a transmission request from a client terminal and the second medical support screen of the medical staff member designated by the transmission request and transmits the first medical support screen or the second medical support screen to the client terminal which has transmitted the transmission request. The first medical support screen enables the medical staff to check the communication message while ascertaining a relationship with the patient schedule information. Therefore, the medical staff can rapidly and accurately ascertain the content of the communication message and smoothly exchange and share information in a team medical treatment. The second medical support screen enables the medical staff to check the communication message while ascertaining relation to the staff schedule information. Therefore, the medical staff can rapidly and accurately ascertain the content of the communication message and smoothly exchange and share information in a team medical treatment.

Among a plurality of communication messages, the communication messages related to the medical staff member designated by the transmission request are displayed on the second medical support screen. According to this structure, the medical staff member can collectively check only the communication messages transmitted to the medical staff member, without performing an operation of searching for the communication messages transmitted to the medical staff member. Therefore, a medical staff member can rapidly perform an operation of checking the communication message and smoothly cooperate with other medical staff members. As a result, the medical staff members smoothly exchange and share information in a team medical treatment.

In addition, the communication messages related to the patient event information and the staff event information are displayed on the first medical support screen and the second medical support screen. Therefore, it is possible to collectively check the communication messages related to the medical procedure that is scheduled to be performed for a patient or the work that is scheduled to be performed by the medical staff. As a result, it is possible to rapidly and accurately ascertain the content of the medical procedure or work.

### BRIEF DESCRIPTION OF THE DRAWINGS

Fig. 1 is a diagram illustrating the structure of a team medical support system according to a first embodiment.
Fig. 2 is a diagram illustrating a table of a patient schedule information storage unit.
Fig. 3 is a diagram illustrating a table of a staff schedule information storage unit.
Fig. 4 is a diagram illustrating a table of a communication message storage unit.
Fig. 5 is a diagram illustrating a table of a patient information storage unit.
Fig. 6 is a diagram illustrating a table of a staff information storage unit.
Fig. 7 is a block diagram schematically illustrating the electrical structure of a computer forming the team medical support device.
Fig. 8 is a functional block diagram illustrating the computer in a case in which a team medical support program starts.
Fig. 9 is a diagram illustrating an operation menu screen.
Fig. 10 is a diagram illustrating a message transmission screen.
Fig. 11 is a diagram illustrating a transmission request screen.
Fig. 12 is a diagram illustrating a transmission request screen on the condition that the transmission of a first medical support screen is requested.
Fig. 13 is a diagram illustrating a transmission request screen on the condition that the transmission of a second medical support screen is requested.
Fig. 14 is a diagram illustrating the first medical support screen.
Fig. 15 is a diagram illustrating a text display state of the first medical support screen.
Fig. 16 is a diagram illustrating the second medical support screen.
Fig. 17 is a diagram illustrating a text display state of the second medical support screen.
Fig. 18 is a flowchart illustrating a medical support screen transmission process.
Fig. 19 is a diagram illustrating the structure of a team medical support system according to a second embodiment.
Fig. 20 is a diagram illustrating a table of a communication message storage unit according to the second embodiment.
Fig. 21 is a diagram illustrating a first medical support screen according to the second embodiment.
Fig. 22 is a diagram illustrating the display state of patient-related messages on the first medical support screen according to the second embodiment.
Fig. 23 is a diagram illustrating a second medical support screen according to the second embodiment.
Fig. 24 is a diagram illustrating the display state of staff-related messages on the second medical support screen according to the second embodiment.
Fig. 25 is a flowchart illustrating a medical support screen transmission process according to the second embodiment.
Fig. 26 is a diagram illustrating the structure of a team medical support system according to a third embodiment.
Fig. 27 is a flowchart illustrating a medical support screen transmission process according to the third embodiment.
Fig. 28 is a diagram illustrating a first medical support screen according to the third embodiment.
Fig. 29 is a diagram illustrating the structure of a team medical support system according to a fourth embodiment.
Fig. 30 is a flowchart illustrating a communication message reception process according to the fourth embodiment.
Fig. 31 is a diagram illustrating the notification state of a communication message according to the fourth embodiment.
Fig. 32 is a diagram illustrating the structure of a team medical support system according to a fifth embodiment.
Fig. 33 is a flowchart illustrating a communication message reception process according to the fifth embodiment.
Fig. 34 is a diagram illustrating the structure of a team medical support system according to a sixth embodiment.
Fig. 35 is a flowchart illustrating a communication message reception process according to the sixth embodiment.

### DESCRIPTION OF THE PREFERRED EMBODIMENTS

### [First Embodiment]

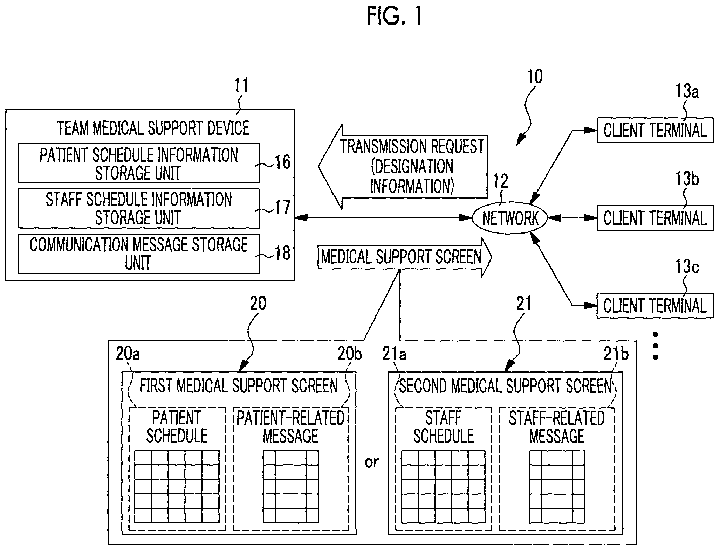

A team medical support system 10 illustrated in Fig. 1 is a client-server computer system that supports a team medical treatment in which a medical team including a plurality of medical staff members, such as doctors or nurses, examines patients and enables the medical staff members to smoothly exchange and share information therebetween. The team medical support system 10 comprises a team medical support device 11 that is installed in, for example, an information processing room of a medical institution, such as a hospital, and a plurality of client terminals 13a to 13c that are connected to the team medical support device 11 through a network 12. The client terminals 13a to 13c are desktop or notebook personal computers or portable terminals, such as mobile phones, smart phones, or tablet terminals, and are used by the medical staff in the medical institution. The network 12 is a local area network (LAN) that is constructed in the medical institution.

The team medical support device 11 comprises a patient schedule information storage unit 16, a staff schedule information storage unit 17, and a communication message storage unit 18. The patient schedule information storage unit 16 stores patient schedule information indicating a plan for patient examination which is generally called a clinical path. The plan for patient examination is a plan for a medical procedure, such as an examination, a cure, or a medical treatment, which is scheduled to be performed for a patient. Patient event information indicating a medical procedure, such as an examination, a cure or a medical treatment, is registered in the patient schedule information.

The staff schedule information storage unit 17 stores staff schedule information indicating a work schedule of the medical staff who is in charge of patients. Staff event information indicating the relationship between the patient and work that the medical staff is in charge of is registered in the staff schedule information. The communication message storage unit 18 stores a plurality of communication messages, such as messages related to a patient, which are exchanged between the medical staff members through the client terminals 13a to 13c.

In a case in which a medical support screen transmission request is received from the client terminals 13a to 13c, the team medical support device 11 creates a medical support screen on the basis of the patient schedule information, the staff schedule information, and the communication message. The medical support screen is used to support medical staff members such that the medical staff members smoothly exchange and share information and includes a first medical support screen 20 on which the patient schedule information of a patient designated by designation information that is included in the transmission request and communication messages related to the patient are displayed and a second medical support screen 21 on which the staff schedule information of a medical staff member designated by the designation information that is included in the transmission request and communication messages related to the medical staff member are displayed.

A patient schedule display region 20a and a patient-related message display region 20b are displayed in parallel on the first medical support screen 20. The patient schedule information of one patient designated by the transmission request is displayed in the patient schedule display region 20a in the form of a table in which patient event information is arranged in chronological order. Communication messages which are related to the patient designated by the transmission request among a plurality of communication messages are summarized and displayed in the form of a table in the patient-related message display region 20b.

A staff schedule display region 21a and a staff-related message display region 21b are displayed in parallel on the second medical support screen 21. The staff schedule information of one medical staff member designated by the transmission request is displayed in the staff schedule display region 21a in the form of a table in which staff event information is arranged in chronological order. Communication messages which are related to the medical staff member designated by the transmission request among a plurality of communication messages are summarized and displayed in the form of a table in the staff-related message display region 21b.

The team medical support device 11 transmits the first medical support screen 20 or the second medical support screen 21 created in response to the transmission request to the client terminals 13a to 13c which have transmitted the transmission request. The medical support screen transmitted to the client terminals 13a to 13c is displayed on displays of the client terminals 13a to 13c. Therefore, the medical staff can refer to the medical support screen and can smoothly exchange and share information required for a team medical treatment.

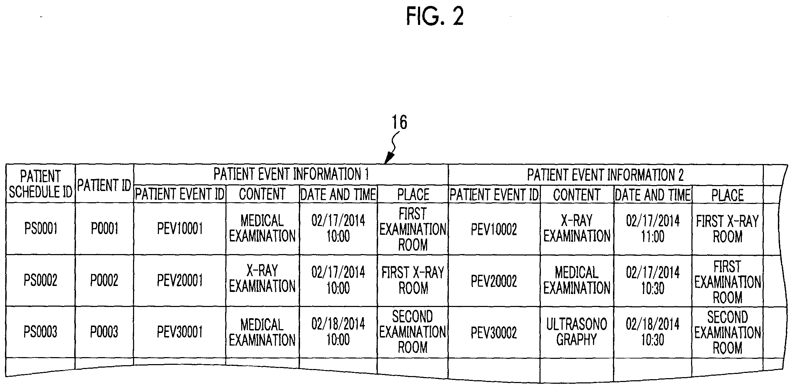

As illustrated in Fig. 2, the patient schedule information storage unit 16 is, for example, a table-type database in which the patient schedule information of each patient is stored in each record. A patient schedule ID, a patient ID, and patient event information are stored in each record of the patient schedule information storage unit 16. The patient schedule ID is an ID for identifying patient schedule information and includes letters "PS" and an identification number. The patient ID is an ID for identifying a patient and includes a letter "P" and an identification number. The patient event information includes a patient event ID, the content of a medical procedure, a date and time, and a place. The patient event ID includes letters "PEV" and an identification number.

For example, the patient schedule information of a patient with a patient ID "P0001" is stored in a record corresponding to a patient schedule ID "PS0001" and a patient event ID "PEV10001", content "medical examination" , a date and time "2014/02/17 10 : 00", and a place "first examination room" are registered in patient event information 1. In addition, the patient schedule information of a patient with a patient ID "P0003" is stored in a record corresponding to a patient schedule ID "PS0003" and a patient event ID "PEV20002", content "ultrasonography", a date and time "2014/02/18 10:30", and a place "first examination room" are registered in patient event information 2.

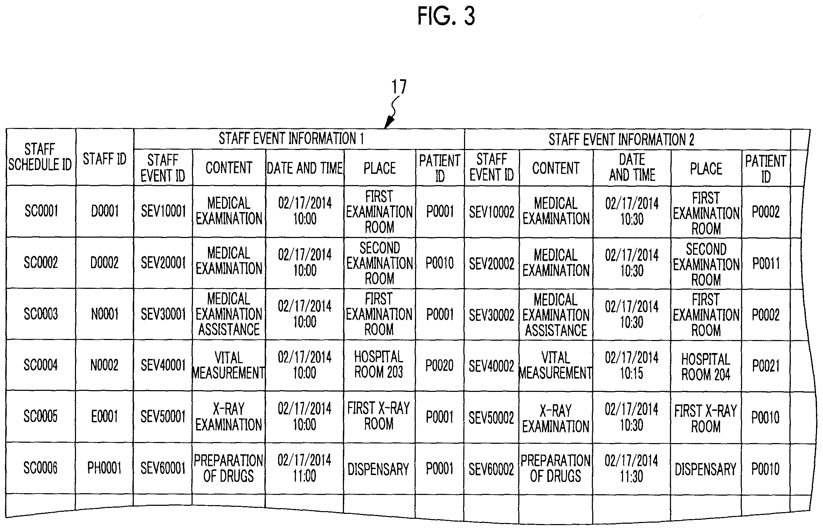

As illustrated in Fig. 3, the staff schedule information storage unit 17 is, for example, a table-type database in which the staff schedule information of each medical staff member is stored in each record. A staff schedule ID, a staff ID, and staff event information are stored in each record of the staff schedule information storage unit 17.

The staff schedule ID is an ID for identifying staff schedule information and includes letters "SC" and an identification number. The staff ID is an ID for identifying a medical staff and includes letters indicating the job type of the medical staff and an identification number. For the letters indicating the job type of the medical staff, for example, "D" indicates a doctor, "N" indicates a nurse, "E" indicates an X-ray or computed tomography (CT) technician, and "PH" indicates a pharmacist. The staff event information includes a staff event ID, the content of a work, a date and time, a place, and a patient ID of a patient who is scheduled to be subjected to a treatment. The staff event ID includes letters "SEV" and an identification number.

For example, the staff schedule information of a doctor with a staff ID "D0001" is stored in a record corresponding to a staff schedule ID "SC0001" and a staff event ID "SEV10001", content "medical examination" , a date and time "2014/02/17 10:00", a place "first examination room", and a patient ID "P0001" are registered in staff event information 1. In addition, the staff schedule information of a nurse with a staff ID "N0001" is stored in a record corresponding to a staff schedule ID "SC0003" and a staff event ID "SEV30001", content "medical examination assist", a date and time "2014/02/17 10:00", a place "first examination room", and a patient ID "P0001" are registered in staff event information 1.

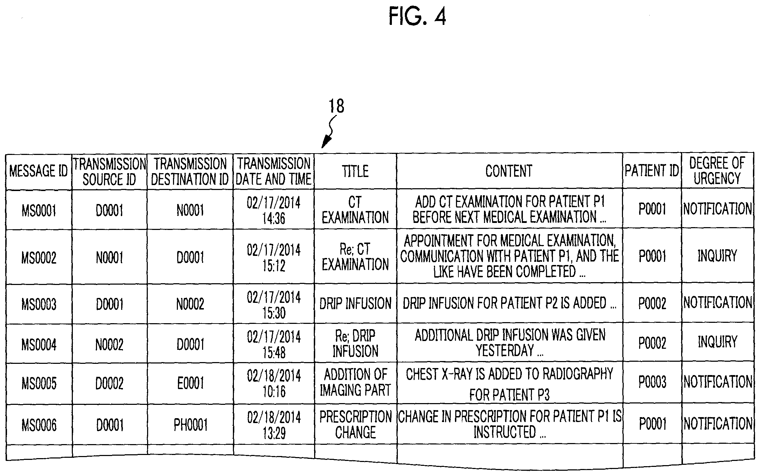

As illustrated in Fig. 4, the communication message storage unit 18 is, for example, a table-type database in which each communication message exchanged among the client terminals 13a to 13c is stored in each record. A message ID, a transmission source ID, a transmission destination ID, a transmission date and time, a title, text, a patient ID, and the degree of urgency are registered in each record of the communication message storage unit 18.

The message ID is an ID for identifying a communication message and includes letters "MS" and an identification number. The transmission source ID and the transmission destination ID are a staff ID of a medical staff member who has transmitted a communication message and a staff ID of a medical staff member who is the destination of the communication message, respectively. The patient ID is an ID of the patient related to the communication message. The degree of urgency is the degree of urgency of a communication message and is represented by, for example, three levels, that is, a "notification", an "instruction", and an "inquiry". The "notification" indicates the highest degree of urgency, followed by the "instruction" and the "inquiry" in this order.

For example, information related to a communication message transmitted from a doctor to a nurse is stored in a record corresponding to a message ID "MS0001". A transmission source ID "D0001", a transmission destination ID "N0001" , a transmission date and time "2014/02/17 14:36", a title "CT examination", content "a CT examination for a patient P1 is added before a next medical examination ...", a patient ID "P0001", and the degree of urgency "notification" are registered in the record corresponding to "MS0001". In addition, a reply to a communication message with a message ID "MS0001" is stored in a record corresponding to a message ID "MS0002". A transmission source ID "N0001", a transmission destination ID "D0001", a transmission date and time "2014/02/17 15:12", a title "Re; CT examination", content "examination reservation and communication with the patient P1 have been completed ...", a patient ID "P0001", and the degree of urgency "notification" are registered in the record corresponding to "MS0002".

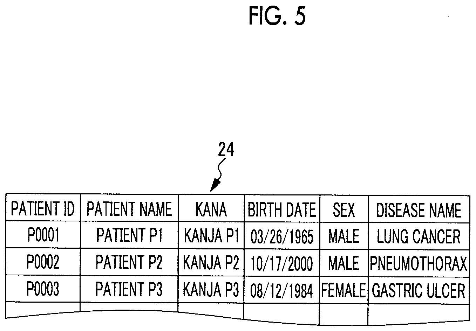

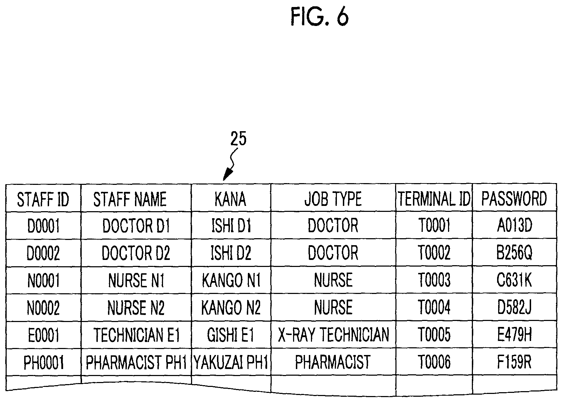

As illustrated in Figs. 5 and 6, the team medical support device 11 comprises a patient information storage unit 24 that stores the personal information of patients and a staff information storage unit 25 that stores the personal information of the medical staff, in addition to the patient schedule information storage unit 16, the staff schedule information storage unit 17, and the communication message storage unit 18. In a condition of creating the first medical support screen 20 and the second medical support screen 21, the team medical support device 11 reads information which is related to patients or the medical staff and has not been stored in the patient schedule information storage unit 16, the staff schedule information storage unit 17, and the communication message storage unit 18 from the patient information storage unit 24 and the staff information storage unit 25.

The patient information storage unit 24 is a table-type database in which the personal information of each patient is registered in each record. For example, a patient ID, a patient name, the Kana of the patient name, a birth date, sex, and a disease name are registered in each record of the patient information storage unit 24.

For example, a patient name "patient P1", Kana "Kanja P1", a birth date "1965/03/26", sex "male", and a disease name "lung cancer" are registered in a record corresponding to a patient ID "P0001". In addition, a patient name "patient P3", Kana "Kanja P3", a birth date "1984/08/12", sex "female", and a disease name "gastric ulcer" are registered in a record corresponding to a patient ID "P0003".

The staff information storage unit 25 is a table-type database in which the personal information of each medical staff member is registered in each record. The personal information of the medical staff includes, for example, a staff ID, a staff name, the Kana of the staff name, a job type, such as a doctor or a nurse, a terminal ID for identifying a client terminal that is used, and a password which is used to log in the team medical support device 11.

For example, a staff name "doctor D1", Kana "Ishi D1", a job type "doctor", a terminal ID "T0001", and a password "A013D" are registered in a record corresponding to the staff ID "D0001". In addition, for example, a staff name "nurse N1", Kana "Kango N1", a job type "nurse", a terminal ID "T0003", and a password "C631K" are registered in a record corresponding to a staff ID "N0001". Similarly, the staff information of an X-ray technician and the staff information of a pharmacist are stored in a record corresponding to a staff ID "E0001" and a record corresponding to a staff ID "PH0001", respectively.

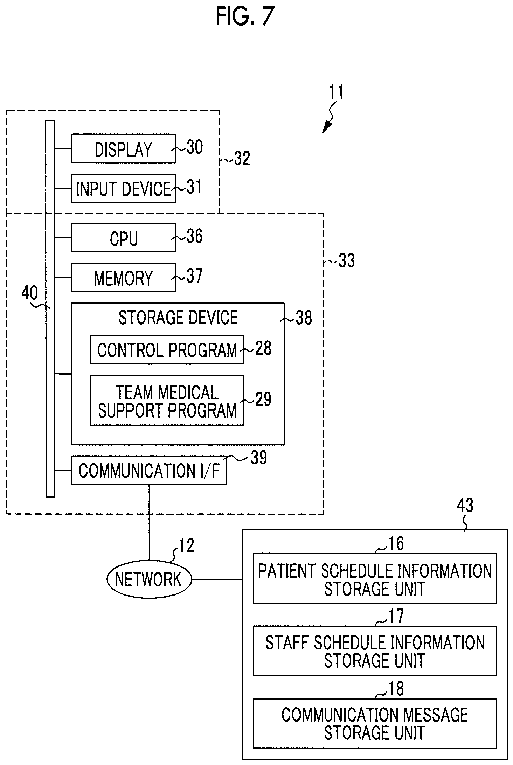

As illustrated in Fig. 7, the team medical support device 11 is configured by installing a control program 28, such as an operating system, or a team medical support program 29, which is an application program that causes a computer to function as the team medical support device 11, in a computer, such as a personal computer or a workstation. The team medical support device 11 comprises a console 32 including a display 30 and an input device 31, such as a touch panel which is integrated with a keyboard, a mouse, and a display, and a main body 33.

The main body 33 of the team medical support device 11 includes a CPU 36, a memory 37, a storage device 38, and a communication I/F 39 which are connected to each other through a data bus 40.

The storage device 38 is a device that stores various kinds of data and is, for example, a hard disk drive. The storage device 38 stores various kinds of programs, such as the control program 28 and the team medical support program 29.

The memory 37 is a work memory that is used by the CPU 36 to perform a process. The CPU 36 loads the control program 28 stored in the storage device 38 to the memory 37 and performs a process according to the program to control the overall operation of each unit of the computer. The communication I/F 39 is a communication interface that is connected to the network 12 and is used to communicate with the client terminals 13a to 13c. In addition, the communication I/F 39 is connected to a data server 43 in which the patient schedule information storage unit 16, the staff schedule information storage unit 17, and the communication message storage unit 18 are constructed through the network 12 such that it can communicate with the data server 43. The data server 43 is an external storage device. Of course, the patient schedule information storage unit 16, the staff schedule information storage unit 17, and the communication message storage unit 18 may be constructed in the storage device 38.

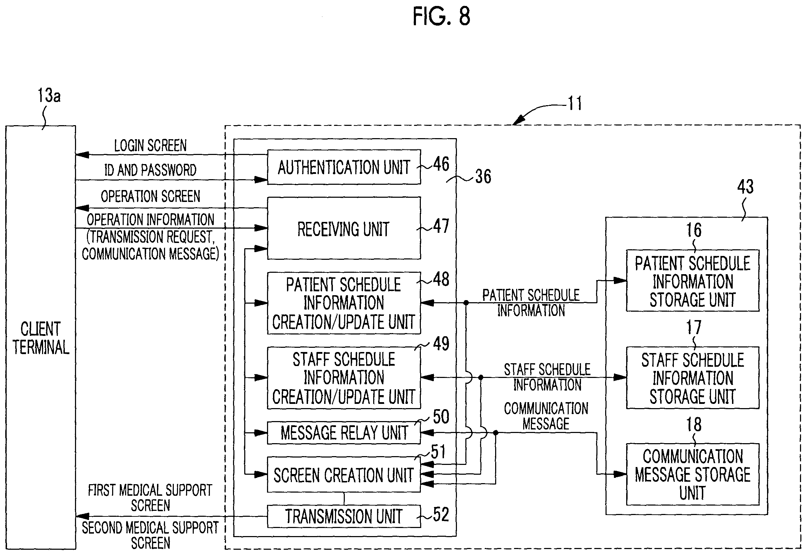

As illustrated in Fig. 8, at the time that the team medical support program 29 starts, the CPU 36 of the team medical support device 11 functions as an authentication unit 46, a receiving unit 47, a patient schedule information creation/update unit 48, a staff schedule information creation/update unit 49, a message relay unit 50, a screen creation unit 51, and a transmission unit 52 in cooperation with, for example, the memory 37.

The authentication unit 46 performs a process of authenticating the client terminals 13a to 13c that have accessed the team medical support device 11. In the authentication process, the authentication unit 46 transmits a login screen that prompts the input of a user ID and a password for login to the client terminals 13a to 13c. The authentication unit 46 compares the user ID and the password input to the login screen with the staff ID and the password stored in the staff information storage unit 25, allows the access of the client terminals 13a to 13c in a case in which the user ID and the password are identical to the staff ID and the password, and does not allow the access in a case in which the user ID and the password are not identical to the staff ID and the password.

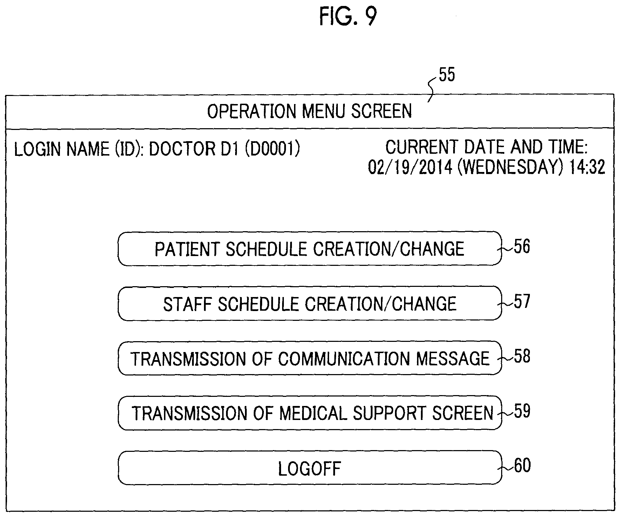

The receiving unit 47 transmits various kinds of operation screens including an operation menu screen 55 illustrated in Fig. 9 to the client terminals 13a to 13c which have been allowed to access the team medical support device by the authentication unit 46 and receives operations which have input through the operation screens of the client terminals 13a to 13c as operation information. The operation menu screen 55 is provided with a plurality of operation menu buttons 56 to 60. In a case in which the client terminals 13a to 13c are personal computers, for example, a mouse is operated to move a pointer on the screen and is clicked to select the operation menu buttons 56 to 60. In a case in which the client terminals 13a to 13c are portable terminals with touch panels, such as smart phones or tablet terminals, the operation menu buttons 56 to 60 are directly selected on the touch panel. At the time that one of the operation menu buttons 56 to 60 of the client terminals 13a to 13c is operated, operation information indicating which of the operation menu buttons is operated is transmitted from the client terminals 13a to 13c to the receiving unit 47.

The "patient schedule creation/change" button 56 of the operation menu screen 55 is operated at the time that patient schedule information is newly created or at the time that the created patient schedule information is changed. At the time that the "patient schedule creation/change" button 56 is operated in the client terminals 13a to 13c, the receiving unit 47 transmits a patient schedule operation screen to the client terminals 13a to 13c. The client terminals 13a to 13c can newly create patient schedule information and change the created patient schedule information through the patient schedule operation screen, which is not illustrated in detail in the drawings. The receiving unit 47 receives the patient schedule information which has been newly created by the client terminals 13a to 13c and the patient schedule information which has been changed by the client terminals 13a to 13c and inputs the information to the patient schedule information creation/update unit 48. The patient schedule information creation/update unit 48 gives a patient schedule ID to the newly created patient schedule information and stores the newly created patient schedule information in the patient schedule information storage unit 16. In addition, for the changed patient schedule information, the patient schedule information creation/update unit 48 rewrites a changed portion in the patient schedule information storage unit 16.

The "staff schedule creation/change" button 57 of the operation menu screen 55 is operated at the time that the staff schedule information is newly created or at the time that the created staff schedule information is changed. At the time that the "staff schedule creation/change" button 57 is operated in the client terminals 13a to 13c, the receiving unit 47 transmits a staff schedule operation screen to the client terminals 13a to 13c. The client terminals 13a to 13c can newly create staff schedule information and change the created staff schedule information through the staff schedule operation screen, which is not illustrated in detail in the drawings. The receiving unit 47 receives the staff schedule information which has been newly created by the client terminals 13a to 13c and the staff schedule information which has been changed by the client terminals 13a to 13c and inputs the information to the staff schedule information creation/update unit 49. The staff schedule information creation/update unit 49 gives a staff schedule ID to the newly created staff schedule information and stores the newly created staff schedule information in the staff schedule information storage unit 17. In addition, for the changed staff schedule information, the staff schedule information creation/update unit 49 rewrites a changed portion in the staff schedule information storage unit 17.

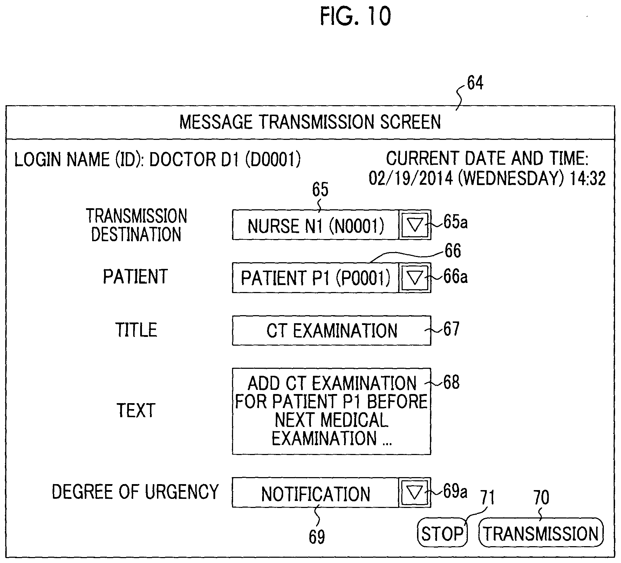

The "communication message transmission" button 58 of the operation menu screen 55 is operated at the time that a communication message is transmitted. At the time that the "communication message transmission" button 58 is operated in the client terminals 13a to 13c, the receiving unit 47 transmits a message transmission screen 64 illustrated in Fig. 10 to the client terminals 13a to 13c.

The message transmission screen 64 includes, for example, a transmission destination designation field 65 for designating the transmission destination of a communication message, a patient designation field 66 for designating a patient corresponding to the communication message, a title input field 67 for inputting the title of the communication message, a text input field 68 for inputting text of the communication message, and an urgency degree designation field 69 for designating the degree of urgency. At the time that list display buttons 65a and 66a which are provided at the right ends of the transmission destination designation field 65 and the patient designation field 66, respectively, are operated, the medical staff list stored in the staff information storage unit 25 and the patient list stored in the patient information storage unit 24 are displayed. A transmission destination and a patient can be selected and designated from the displayed list. In addition, at the time that a list display button 69a which is provided at the right end of the urgency degree designation field 69 is operated, a list of the degrees of urgency is displayed. Therefore, the degree of urgency can be selected and designated from the displayed list.

A transmission button 70 for instructing the transmission of a communication message and a stop button 71 for stopping the transmission of a communication message are provided in a lower right portion of the message transmission screen 64. At the time that the transmission button 70 is operated, the receiving unit 47 receives an input communication message and inputs the communication message to the message relay unit 50. The communication message includes, as information related to a transmission source, the staff ID of a medical staff member who logs in the team medical support device 11 and the transmission date and time, in addition to each kind of information input through the message transmission screen 64. The message relay unit 50 stores the input communication message in the communication message storage unit 18. At the time that the stop button 71 is operated, the display screen of the client terminals 13a to 13c returns from the message transmission screen 64 to the operation menu screen 55.

The message transmission screen 64 indicates a transmission screen of the communication message which is stored in the record corresponding to the message ID "MS0001" in the communication message storage unit 18 illustrated in Fig. 4. "N0001" is input to the transmission destination designation field 65 and "P0001" is input to the patient designation field 66. In addition, a "CT examination" is input to the title input field 67 and a "CT examination for a patient P1 is added before the next medical examination ... " is input to the text input field 68. "Notification" is designated in the urgency degree designation field 69.

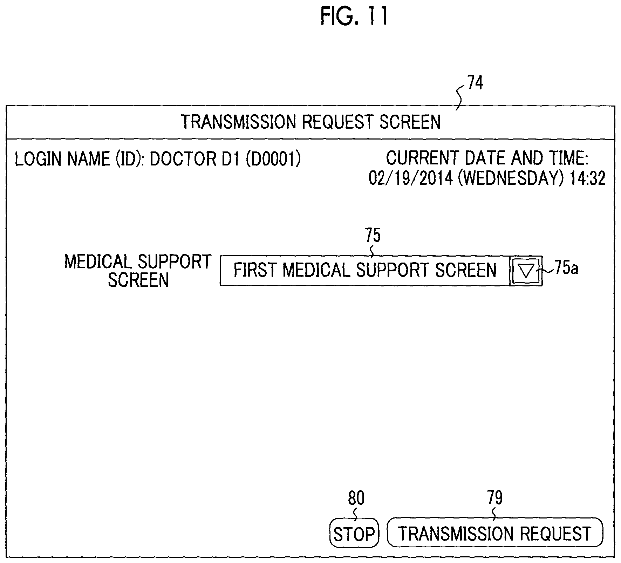

In Fig. 9, the "medical support screen transmission" button 59 of the operation menu screen 55 is operated at the time that there is a request to transmit the first medical support screen 20 (see Fig. 1) on which the information of a designated patient is displayed or the second medical support screen 21 (see Fig. 1) on which the information of a designated medical staff member is displayed. At the time that the "medical support screen transmission" button 59 is operated in the client terminals 13a to 13c, the receiving unit 47 transmits a transmission request screen 74 illustrated in Fig. 11 to the client terminals 13a to 13c. The transmission request screen 74 includes a screen designation field 75 for designating a medical support screen which is required to be transmitted. At the time that a list display button 75a which is provided at the right end of the screen designation field 75 is operated, a list of medical support screens, such as the first medical support screen and the second medical support screen, is displayed. Therefore, a medical support screen can be selected and designated from the displayed list.

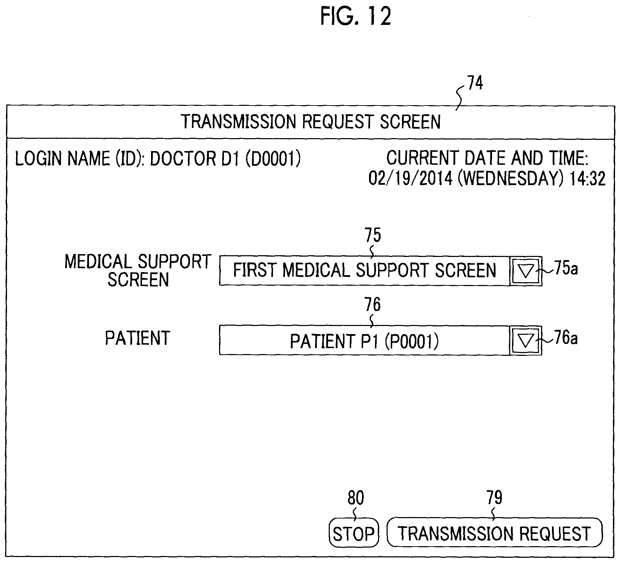

At the time that the first medical support screen is selected in the screen designation field 75 of the transmission request screen 74, a patient designation field 76 is displayed below the screen designation field 75 on the transmission request screen 74, as illustrated in Fig. 12. In a case in which the first medical support screen 20 is designated in the screen designation field 75, the patient designation field 76 is used to designate the patient of which the information is displayed on the first medical support screen 20. At the time that a list display button 76a which is provided at the right end of the patient designation field 76 is operated, a list of the patient stored in the patient information storage unit 24 is displayed. Therefore, a patient can be selected and designated from the displayed list.

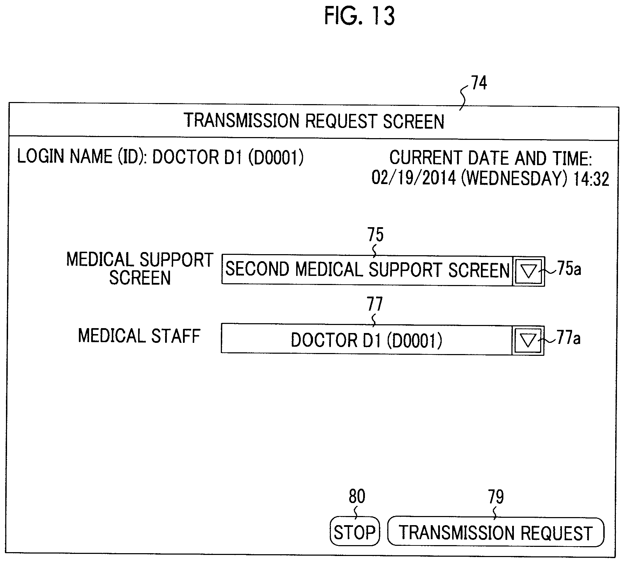

At the time that the second medical support screen is selected in the screen designation field 75 of the transmission request screen 74, a staff designation field 77 is displayed below the screen designation field 75 on the transmission request screen 74, as illustrated in Fig. 13. In a case in which the second medical support screen 21 is designated in the screen designation field 75, the staff designation field 77 is used to designate the medical staff member of which the information is displayed on the second medical support screen 21. At the time that a list display button 77a which is provided at the right end of the staff designation field 77 is operated, a list of the medical staff members stored in the staff information storage unit 25 is displayed. Therefore, a medical staff member can be selected and designated from the displayed list.

A transmission request button 79 for instructing the transmission of a transmission request and a stop button 80 for stopping the transmission of a transmission request are provided in a lower right portion of the transmission request screen 74. At the time that the transmission request button 79 is operated, a transmission request is transmitted as the operation information of the client terminals 13a to 13c to the team medical support device 11. The transmission request includes the patient ID or the staff ID designated in the patient designation field 76 or the staff designation field 77 as designation information for designating a patient or a medical staff member corresponding to the medical support screen. At the time that the stop button 80 is operated, the display screen of the client terminals 13a to 13c returns from the transmission request screen 74 to the operation menu screen 55.

In Fig. 8, in a case in which the creation of the first medical support screen 20 is requested in the transmission request, the screen creation unit 51 reads the patient schedule information of a designated patient from the patient schedule information storage unit 16 and reads a communication message related to the designated patient from the communication message storage unit 18, on the basis of the patient ID designated by the transmission request. The communication message related to the designated patient is a communication message which has content corresponding to the patient designated by the transmission request among a plurality of communication messages. Specifically, the communication message is a communication message in which the patient ID of the patient designated by the transmission request is registered in the communication message storage unit 18 illustrated in Fig. 4. The screen creation unit 51 creates the first medical support screen 20 on the basis of the read patient schedule information and the read communication message.

In a case in which the creation of the second medical support screen 21 is requested in the transmission request, the screen creation unit 51 reads the staff schedule information of a designated medical staff member from the staff schedule information storage unit 17 and reads a communication message related to the designated medical staff member from the communication message storage unit 18, on the basis of the staff ID designated by the transmission request. The communication message related to the designated medical staff member is a communication message which is to be transmitted to the medical staff member designated by the transmission request among a plurality of communication messages. Specifically, the communication message is a communication message in which the staff ID of the medical staff member designated by the transmission request is registered as the transmission destination ID in the communication message storage unit 18 illustrated in Fig. 4. The screen creation unit 51 creates the second medical support screen 21 on the basis of the read staff schedule information and the read communication message.

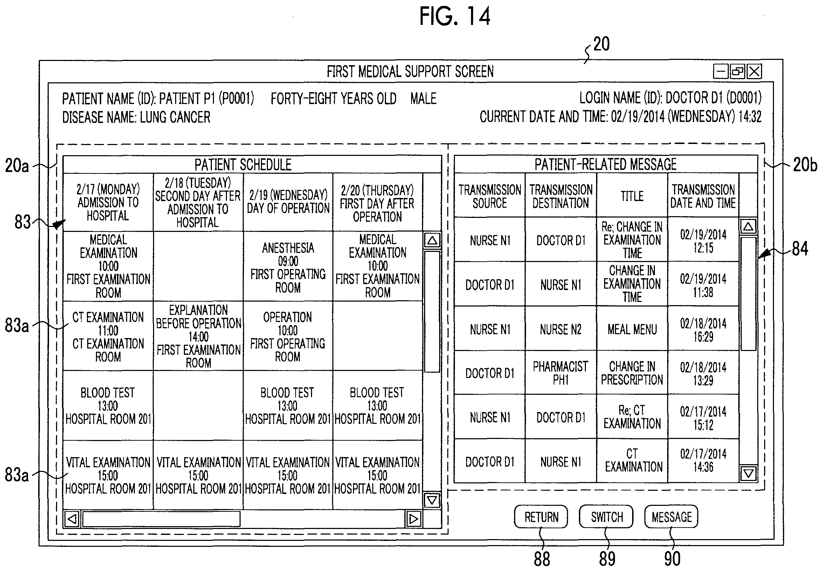

As illustrated in Fig. 14, the patient schedule display region 20a and the patient-related message display region 20b are displayed on the first medical support screen 20 in parallel in the horizontal direction. A patient schedule table 83 in which the patient schedule information read from the patient schedule information storage unit 16 is arranged in the form of a table is displayed in the patient schedule display region 20a which is provided on the left side of the screen. The date and time is allocated in chronological order along the horizontal axis and the vertical axis of the patient schedule table 83 and the patient schedule table 83 comprises a plurality of cells 83a which correspond to the date and time and are arranged in a matrix. The patient event information of the patient schedule information is displayed in each cell 83a.

A patient-related message list 84 in which the communication messages read from the communication message storage unit 18 are summarized in the form of a table is displayed in the patient-related message display region 20b which is provided on the right side of the screen. The transmission source, transmission destination, title, and transmission date and time of a communication message are displayed on the patient-related message list 84 in reverse chronological order from the upper side. In addition, a portion of or the entire text of the communication message may be displayed on the patient-related message list 84, instead of the title or together with the title.

Fig. 14 illustrates a state in which the patient schedule information of the patient P1 and a communication message are displayed on the first medical support screen 20. The patient schedule information of the patient P1 read from the patient schedule information storage unit 16 is displayed in the patient schedule table 83. A communication message related to the patient P1 which is read from the communication message storage unit 18 is displayed in the patient-related message list 84.

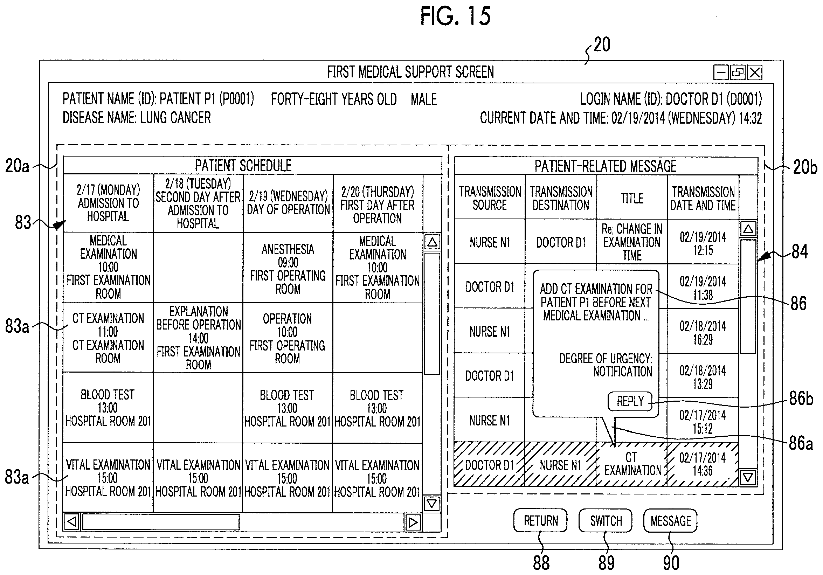

At the time that a communication message is selected from the patient-related message list 84, the background color of the selected communication message is changed and a text display field 86 in which the text of the selected communication message is displayed is displayed so as to be superimposed on the patient-related message list 84, as illustrated in Fig. 15. Since the text display field 86 is connected to the communication message by a leader line 86a, the medical staff can easily understand which communication message the text belongs to. A reply button 86b which is operated at the time that a reply to the communication message displayed in the text display field 86 is made is provided in the text display field 86. At the time that the reply button 86b is operated, the message transmission screen 64 illustrated in Fig. 10 is displayed so as to be superimposed on the first medical support screen 20. On the first medical support screen 20 illustrated in Fig. 15, the text of a title "CT examination" which has been transmitted from a doctor D1 to a nurse N1 is displayed in the text display field 86.

A return button 88, a switching button 89, and a message button 90 are provided below the patient-related message display region 20b on the first medical support screen 20. The return button 88 is operated at the time that the screen returns from the first medical support screen 20 to the operation menu screen 55. The switching button 89 is operated at the time that the screen is switched from the first medical support screen 20 to the second medical support screen 21. At the time that the switching button 89 is operated, a screen for requesting the transmission of the second medical support screen 21 illustrated in Fig. 13 instead of the first medical support screen 20 is displayed. The message button 90 is operated at the time that a communication message is transmitted on the first medical support screen 20. At the time that the message button 90 is operated, the message transmission screen 64 illustrated in Fig. 10 is displayed so as to be superimposed on the first medical support screen 20.

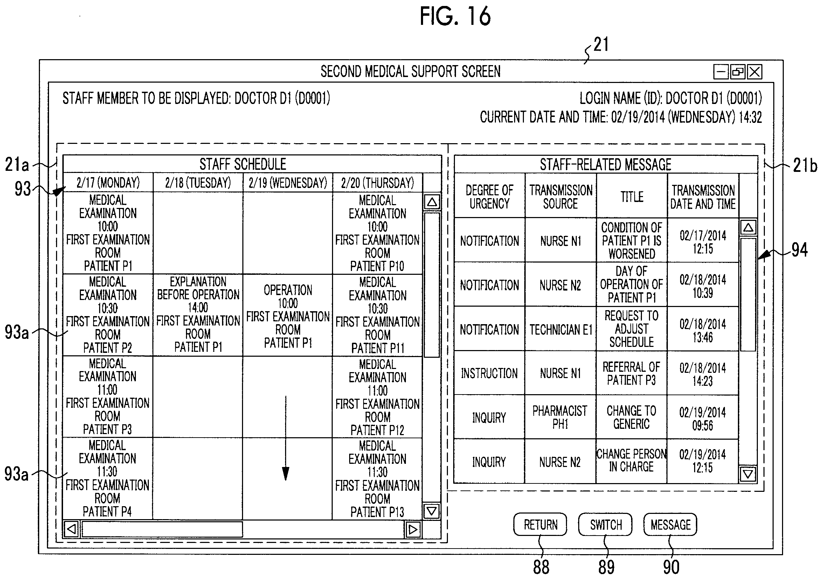

As illustrated in Fig. 16, the staff schedule display region 21a and the staff-related message display region 21b are displayed on the second medical support screen 21 in parallel in the horizontal direction. A staff schedule table 93 in which the staff schedule information read from the staff schedule information storage unit 17 is arranged in the form of a table is displayed in the staff schedule display region 21a which is provided on the left side of the screen. The date and time is allocated in chronological order along the horizontal axis and the vertical axis of the staff schedule table 93 and the staff schedule table 93 comprises a plurality of cells 93a which correspond to the date and time and are arranged in a matrix. The staff event information of the staff schedule information is displayed in each cell 93a.

A staff-related message list 94 in which the communication messages read from the communication message storage unit 18 are summarized in the form of a table is displayed in the staff-related message display region 21b which is provided on the right side of the screen. The degree of urgency, transmission source, title, and transmission date and time of a communication message are displayed on the staff-related message list 94 in descending order of the degree of urgency from the upper side. In addition, in a case in which there are a plurality of communication messages having the same degree of urgency, the communication messages are displayed in reverse chronological order of the transmission date and time from the upper side. In addition, a portion of or the entire text may be displayed on the staff-related message list 94, instead of the title or together with the title.

Fig. 16 illustrates a state in which the staff schedule information of the doctor D1 and a communication message are displayed on the second medical support screen 21. The staff schedule information of the doctor D1 read from the staff schedule information storage unit 17 is displayed in the staff schedule table 93. A communication message transmitted to the doctor D1 which has been read from the communication message storage unit 18 is displayed in the staff-related message list 94.

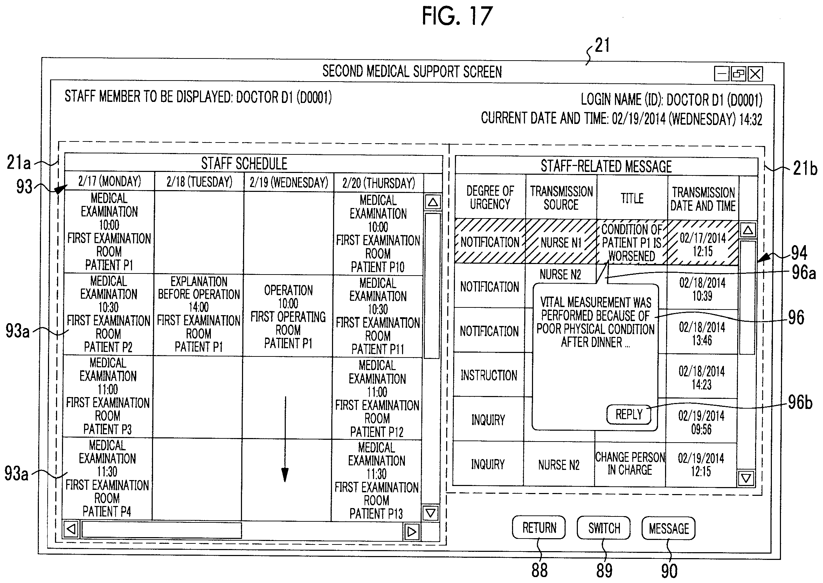

At the time that a communication message is selected from the staff-related message list 94, the background color of the selected communication message is changed and a text display field 96 in which the text of the selected communication message is displayed is displayed so as to be superimposed on the staff-related message list 94, as illustrated in Fig. 17. Similarly to the first medical support screen 20, since the text display field 96 is connected to the communication message by a leader line 96a, the medical staff can easily understand which communication message the text belongs to. Similarly to the first medical support screen 20, a reply button 96b is provided in the text display field 96. On the second medical support screen 21 illustrated in Fig. 17, the text of a title "the condition of the patient P1 is worsened" which has been transmitted from the nurse N1 to the doctor D1 is displayed in the text display field 96.

Similarly to the first medical support screen 20, a return button 88, a switching button 89, and a message button 90 are provided below the staff-related message display region 21b on the second medical support screen 21.

The transmission unit 52 transmits the first medical support screen 20 or the second medical support screen 21 which has been created by the screen creation unit 51 on the basis of the transmission request to the client terminals 13a to 13c which have transmitted the transmission request.

The "log-off" button 60 of the operation menu screen 55 is operated at the time that the user logs off the team medical support device 11. At the time that the "log-off" button 60 is operated in the client terminals 13a to 13c, the authentication unit 46 cancels the authenticated state of the client terminals 13a to 13c.

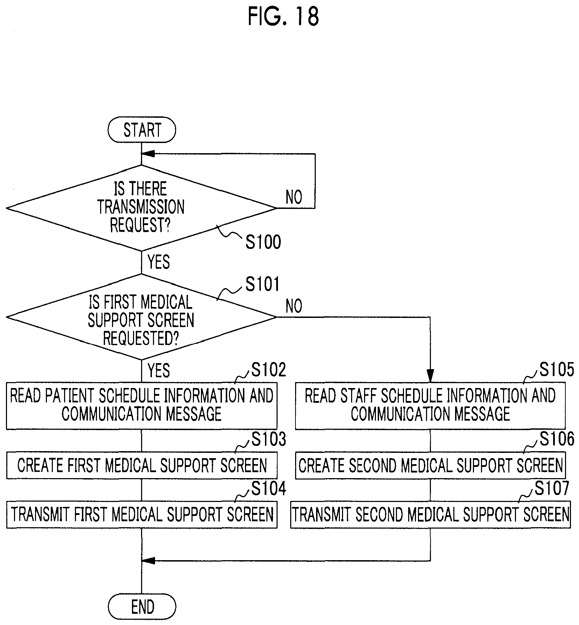

Next, the operation of the above-mentioned structure will be described with reference to a flowchart illustrated in Fig. 18. The team medical support device 11 waits for a medical support screen transmission request from the client terminals 13a to 13c (S100). At the time that a transmission request from the client terminals 13a to 13c is received (YES in S100), the screen creation unit 51 specifies the type of medical support screen corresponding to the transmission request (S101).

In a case in which the transmission of the first medical support screen 20 is requested in the transmission request (YES in S101), the screen creation unit 51 reads the patient schedule information of a patient designated by the transmission request from the patient schedule information storage unit 16 and reads a communication message related to the designated patient from the communication message storage unit 18 (S102).

The screen creation unit 51 creates the first medical support screen 20 on the basis of the read patient schedule information and the read communication message (S103). At the time that the creation of the first medical support screen 20 is completed, the transmission unit 52 transmits the first medical support screen 20 to the client terminals 13a to 13c which have transmitted to the transmission request (S104).

The client terminals 13a to 13c which have received the first medical support screen 20 display the first medical support screen 20 on the displays. The patient schedule display region 20a is provided on the left side of the first medical support screen 20 and the patient-related message display region 20b is provided on the right side of the first medical support screen 20. The patient schedule table 83 in which the patient schedule information read from the patient schedule information storage unit 16 is arranged in the form of a table is displayed in the patient schedule display region 20a. In the patient schedule table 83, the event information of the patient schedule information is displayed for each date which is arranged in the horizontal axis in chronological order from the upper side. The patient-related message list 84 in which the communication messages read from the communication message storage unit 18 are summarized in the form of a table is displayed in the patient-related message display region 20b. In the patient-related message list 84, the transmission source, transmission destination, title, and transmission date and time of a communication message are displayed in reverse chronological order of the transmission date and time from the upper side.

At the time that a communication message is selected from the patient-related message list 84, the text display field 86 in which the text of the selected communication message is displayed is displayed so as to be superimposed on the patient-related message list 84. The text display field 86 is connected to the communication message by the leader line 86a.

In a case in which the transmission of the second medical support screen 21 is requested in the transmission request (NO in S101), the screen creation unit 51 reads the staff schedule information of a medical staff member designated by the transmission request from the staff schedule information storage unit 17 and reads a communication message related to the designated medical staff member from the communication message storage unit 18 (S105).

The screen creation unit 51 creates the second medical support screen 21 on the basis of the read staff schedule information and the read communication message (S106). At the time that the creation of the second medical support screen 21 is completed, the transmission unit 52 transmits the second medical support screen 21 to the client terminals 13a to 13c which have transmitted to the transmission request (S107).

The staff schedule display region 21a is provided on the left side of the second medical support screen 21 and the staff-related message display region 21b is provided on the right side of the second medical support screen 21. The staff schedule table 93 in which the staff schedule information read from the staff schedule information storage unit 17 is arranged the form of a table is displayed in the staff schedule display region 21a. In the staff schedule table 93, the event information of the patient schedule information is displayed for each date which is arranged in the horizontal axis in chronological order from the upper side. The staff-related message list 94 in which the communication messages read from the communication message storage unit 18 are summarized in the form of a table is displayed in the staff-related message display region 21b. In the staff-related message list 94, the degree of urgency, transmission source, title, and transmission date and time of a communication message are displayed in descending order of the degree of urgency from the upper side.

At the time that a communication message is selected from the staff-related message list 94, the text display field 96 in which the text of the selected communication message is displayed is displayed on the staff-related message list 94, similarly to the first medical support screen 20. The text display field 96 is connected to the communication message by the leader line 96a, similarly to the first medical support screen 20.

As described above, the team medical support device 11 according to the invention creates the first medical support screen 20 on which the information of the patient designated by a transmission request is displayed and the second medical support screen 21 on which the information of the medical staff member designated by the same transmission request is displayed, in response to the transmission request from the client terminals 13a to 13c used by the medical staff, and transmits the first medical support screen 20 or the second medical support screen 21 to the client terminals 13a to 13c which have transmitted the transmission request. The first medical support screen 20 enables the medical staff to check the communication message related to a patient while checking relation to the patient schedule information. Therefore, the medical staff can rapidly and accurately ascertain the content of the communication message and smoothly exchange and share information in a team medical treatment. The second medical support screen 21 enables the medical staff to check the communication message transmitted to the medical staff while checking the relation between the communication message and the staff schedule information. Therefore, the medical staff can rapidly and accurately ascertain the content of the communication message and smoothly exchange and share information in a team medical treatment.

On the second medical support screen 21, among a plurality of communication messages, the communication messages related to the medical staff of which the staff schedule information is displayed is summarized and displayed in the form of a table. According to this structure, the medical staff can collectively check only the communication messages transmitted to the medical staff, without performing an operation of searching for the communication messages transmitted to the medical staff. Therefore, a medical staff member can rapidly perform an operation of checking the communication message and smoothly cooperate with other medical staff members. Therefore, the medical staff members smoothly exchange and share information in a team medical treatment.

The transmission source, transmission destination, title, and transmission date and time of a communication message are displayed in the patient-related message list 84 of the first medical support screen 20. Therefore, it is possible to rapidly ascertain the outline of the communication message only from the displayed content. In addition, since the communication messages are arranged in reverse chronological order of the transmission date and time, it is possible to easily and accurately check the time-series relationship between the communication messages.

The degree of urgency, transmission source, title, and transmission date and time of a communication message are displayed in the staff-related message list 94 of the second medical support screen 21. Therefore, it is possible to rapidly ascertain the outline of the communication message or the degree of urgency only from the displayed content. In addition, since the communication messages are arranged in descending order of the degree of urgency, it is possible to preferentially check a communication message with a high degree of urgency.

In the above-described embodiment, as the communication message related to a designated patient, a communication message in which the patient ID is designated at the time that the communication message is transmitted is displayed in the patient-related message display region. However, the communication messages related to the designated patient may include a communication message in which the name of the designated patient is described in the text of the communication message. In addition, as the communication message related to the medical staff, the communication message transmitted to a designated medical staff member is displayed. However, for example, the communication messages related to the medical staff may include a communication message of which the transmission source is the designated medical staff and a communication message in which the name of the designated medical staff is described in the text of the message.

Next, second to sixth embodiments of the invention will be described. The same structures as those in the first embodiment are denoted by the same reference numerals and the detailed description thereof will not be repeated.

### [Second Embodiment]

In the first embodiment, the communication messages related to the patient and the medical staff member designated by the transmission request are summarized and displayed in the patient-related message display region 20b and the staff-related message display region 21b, respectively. Therefore, it is possible to collectively check communication messages related to the entire plan for examining a patient or the entire work schedule of the medical staff member. However, in a team medical treatment, in many cases, in addition to the communication messages related to the entire plan for examining a patient or the entire work schedule of the medical staff, communication messages related to a specific medical procedure for a patient or a specific work of the medical staff are exchanged between the medical staff members. Therefore, in a team medical field, there is a demand for collectively checking the communication messages related to a medical procedure for a patient or the work of the medical staff. This embodiment provides a technique which can collectively check communication messages related to a medical procedure for a patient or the work of the medical staff. Therefore, information is smoothly exchanged and shared in the team medical treatment.

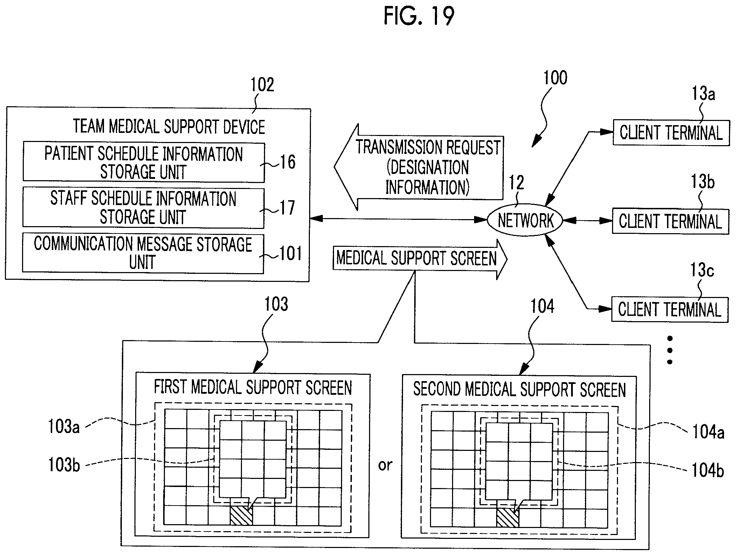

As illustrated in Fig. 19, a team medical support system 100 according to this embodiment comprises a team medical support device 102 that comprises a communication message storage unit 101, instead of the communication message storage unit 18 according to the first embodiment. The team medical support device 102 creates a first medical support screen 103 and a second medical support screen 104 which have different screen structures from those in the first embodiment and transmits the first medical support screen 103 and the second medical support screen 104, in response to a transmission request from client terminals 13a to 13c.

The first medical support screen 103 comprises a patient schedule display region 103a in which patient schedule information of a patient designated by designation information of the transmission request is displayed and a patient-related message display region 103b in which communication messages related to patient event information selected in the patient schedule display region 103a are summarized and displayed. The second medical support screen 104 comprises a staff schedule display region 104a in which staff schedule information of a medical staff member designated by the designation information of the transmission request is displayed and a staff-related message display region 104b in which communication messages related to staff event information selected in the staff schedule display region 104a are summarized and displayed.

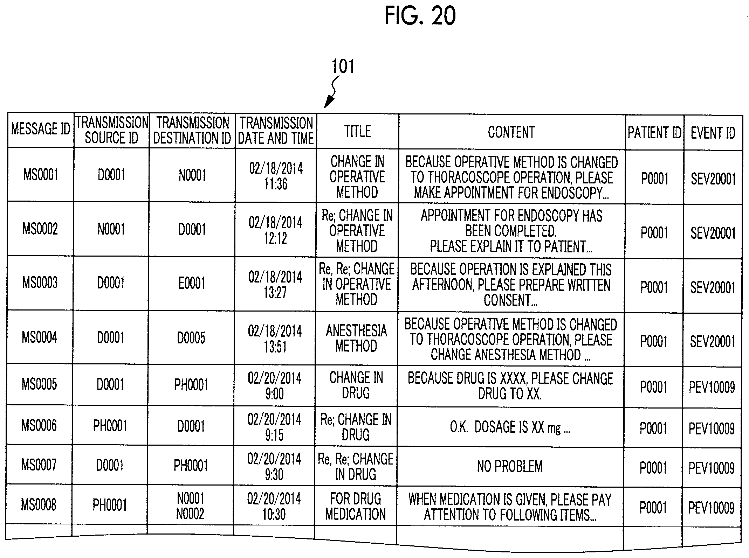

As illustrated in Fig. 20, similarly to the communication message storage unit 18, a message ID, a transmission source ID, a transmission destination ID, a transmission date and time, a title, text, and a patient ID are registered in each record of the communication message storage unit 101. In addition, an event ID is registered in each record of the communication message storage unit 101, unlike the communication message storage unit 18. A patient event ID of the patient event information related to the communication message or a staff event ID of the staff event information related to the communication message is registered in the event ID.

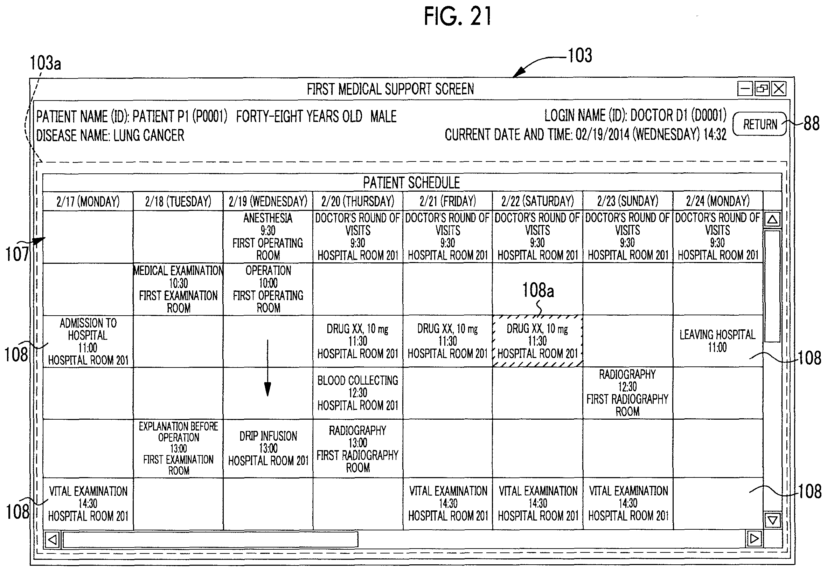

In a case in which a request to transmit the first medical support screen 103 is received, a screen creation unit 51 (see Fig. 8) of the team medical support device 102 creates the first medical support screen 103 including only the patient schedule display region 103a, as illustrated in Fig. 21. A patient schedule table 107 in which patient schedule information is arranged in the form of a table is arranged in the patient schedule display region 103a. The date and time is allocated in chronological order along the horizontal axis and the vertical axis of the patient schedule table 107 and the patient schedule table 107 comprises a plurality of cells 108 which correspond to the date and time and are arranged in a matrix. The patient event information of the patient schedule information is displayed in each cell 108. A transmission unit 52 transmits the first medical support screen 103 which has been created in response to the transmission request to the client terminals 13a to 13c which have transmitted the transmission request.

In a case in which the communication messages related to the patient event information are collectively checked, a cell 108 which is displayed in the patient schedule table 107 is selected in the client terminals 13a to 13c. At the time that patient event information is selected by the selection of the cell 108, patient event selection information indicating that the patient event information has been selected is transmitted as operation information of the client terminals 13a to 13c to the team medical support device 102. The patient event selection information includes an event ID of the selected patient event information. For example, the background color of the cell 108a in which the selected patient event information is displayed is changed such that the medical staff can recognize the selected state of the cell 108a.

In a case in which a receiving unit 47 receives the patient event selection information, the screen creation unit 51 of the team medical support device 102 reads a communication message related to the selected patient event information, specifically, a communication message in which the same patient event ID as that included in the patient event selection information is recorded, from the communication message storage unit 101, and re-creates the first medical support screen 103 having the patient-related message display region 103b.

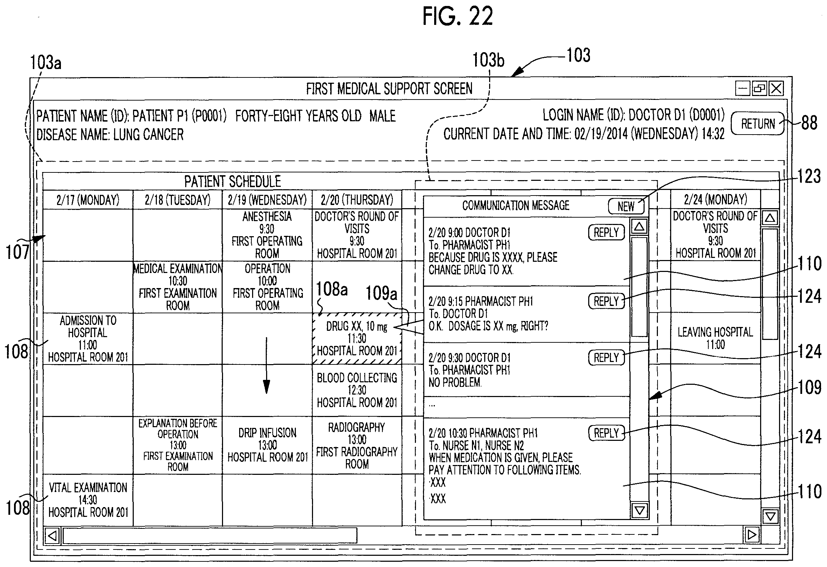

As illustrated in Fig. 22, a patient-related message display region 103b is provided on the first medical support screen 103 which has been re-created by the selection of the patient event information so as to be superimposed on the patient schedule display region 103a. A patient message list 109 in which communication messages related to the selected patient event information are summarized and displayed in the form of a table is displayed in the patient-related message display region 103b. Therefore, on the first medical support screen 103, the patient schedule information and the patient-related messages are displayed in parallel. The patient message list 109 comprises a plurality of message display fields 110. For example, the transmission date and time, transmission source, transmission destination, and text of the communication message are displayed in each message display field 110 in the order of the transmission date and time. In addition, the patient message list 109 is connected to the cell 108 by a leader line 109a such that the relation of the patient message list 109 to the selected patient event information can be easily recognized.

For example, in a case in which patient event information in a cell 108a is selected in the patient schedule table 107, the screen creation unit 51 reads a communication message related to the selected patient event information from the communication message storage unit 101. For example, in a case in which the patient event ID of the patient event information in the cell 108a is "PEV10009", "MS0005" to "MS0008" in which "PEV10009" is registered in the event ID are read from the communication message storage unit 101 illustrated in Fig. 20. The screen creation unit 51 re-creates the first medical support screen 103 in which the transmission date and time, transmission source, transmission destination, and text of the communication messages "MS0005" to "MS0008" are displayed in each message display field 110 of the patient message list 109.

The transmission unit 52 transmits the re-created first medical support screen 103 to the client terminals 13a to 13c. Since the first medical support screen 103 is displayed on displays of the client terminals 13a to 13c, the medical staff can collectively check the communication messages related to a medical procedure for a patient.

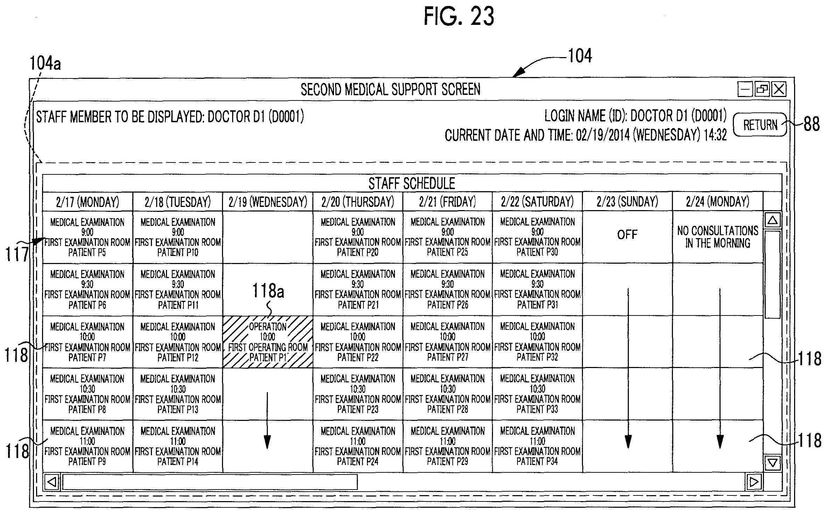

In a case in which a request to transmit the second medical support screen 104 is received, the screen creation unit 51 of the team medical support device 102 creates the second medical support screen 104 including only the staff schedule display region 104a, as illustrated in Fig. 23. A staff schedule table 117 in which staff schedule information is arranged in the form of a table is arranged in the staff schedule display region 104a. The date and time is allocated in chronological order along the horizontal axis and the vertical axis of the staff schedule table 117 and the staff schedule table 117 comprises a plurality of cells 118 which correspond to the date and time and are arranged in a matrix. The staff event information of the staff schedule information is displayed in each cell 118. The transmission unit 52 transmits the second medical support screen 104 which has been created in response to the transmission request to the client terminals 13a to 13c which have transmitted the transmission request.

In a case in which the communication messages related to the staff event information are collectively checked, a cell 118 which is displayed in the staff schedule table 117 is selected in the client terminals 13a to 13c, similarly to the first medical support screen 103. At the time that staff event information is selected by the selection of the cell 118, staff event selection information is transmitted from the client terminals 13a to 13c to the team medical support device 102. The staff event selection information includes an event ID of the selected staff event information. For example, the screen creation unit 51 reads a communication message in which the same staff event ID as that of the staff event selection information is registered in a record from the communication message storage unit 101 and re-creates the second medical support screen 104 having the staff-related message display region 104b.

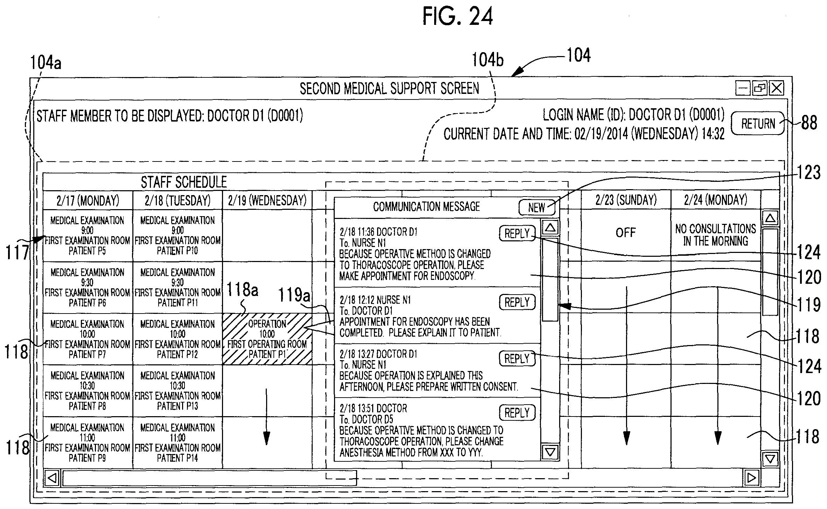

As illustrated in Fig. 24, a staff-related message display region 104b is provided on the second medical support screen 104 which has been re-created by the selection of the staff event information so as to be superimposed on the staff schedule display region 104a. A staff message list 119 in which communication messages related to the selected staff event information are summarized and displayed in the form of a table is displayed in the staff-related message display region 104b. Therefore, on the second medical support screen 104, the staff schedule information and the staff-related messages are displayed in parallel. In the staff message list 119, similarly to the patient message list 109, for example, the transmission date and time, transmission source, transmission destination, and text of the communication message are displayed in each message display field 120 in the order of the transmission date and time. The staff message list 119 is connected to the cell 118 by a leader line 119a such that the relation of the staff message list 119 to the selected staff event information can be easily recognized.

For example, in a case in which staff event information in a cell 118a is selected in the staff schedule table 117, the screen creation unit 51 reads a communication message related to the selected staff event information from the communication message storage unit 101. For example, in a case in which the staff event ID of the staff event information in the cell 118a is "SEV20001", "MS0001" to "MS0004" in which "SEV20001" is registered in the event ID are read from the communication message storage unit 101 illustrated in Fig. 20. The screen creation unit 51 re-creates the second medical support screen 104 in which the transmission date and time, transmission source, transmission destination, and text of the communication messages "MS0001" to "MS0004" are displayed in each message display field 120 of the staff message list 119.

The transmission unit 52 re-transmits the re-created second medical support screen 104 to the client terminals 13a to 13c. Since the second medical support screen 104 is displayed on the displays of the client terminals 13a to 13c, the medical staff can collectively check the communication messages related to the work of the medical staff.

A new button 123 and a reply button 124 are provided in each of the patient message list 109 and the staff message list 119. The new button 123 is arranged in a title portion in which a name is displayed in the patient message list 109 and the staff message list 119 and is operated in a case in which communication messages related to the selected patient event information and the selected staff event information are newly transmitted. The reply button 124 is arranged in each of the message display fields 110 and 120 of the patient message list 109 and the staff message list 119 and is operated in a case in which a reply to the communication message displayed in each of the message display fields 110 and 120 is made.

In Fig. 8, in a case in which the receiving unit 47 receives operation information indicating the operation of the new button 123 or the reply button 124, a message transmission screen is transmitted to the client terminals 13a to 13c which have transmitted the operation information. The message transmission screen (not illustrated) which is transmitted in a case in which the new button 123 is operated differs from the message transmission screen 64 illustrated in Fig. 10 in that it does not include the patient designation field 66 and comprises the transmission destination designation field 65, the title input field 67, and the text input field 68. In a case in which a communication message is transmitted on the basis of the patient event information and the staff event information, the patient designation field 66 can be omitted since the patient related to the communication message is known in advance.

At the time that each information item is input to the message transmission screen and a transmission button is operated, a communication message including a transmission source ID, a transmission date and time, a target patient ID, and a patient event ID or a staff event ID, in addition to each information item input through the message transmission screen, is transmitted from the client terminals 13a to 13c to the team medical support device 102. The transmission source ID, the patient ID, and the patient event ID or the staff event ID are specified on the basis of the log-in ID which is used to log in the team medical support device 102, the patient ID designated by the transmission request, and the event ID of the selected patient event information or the selected staff event information. The team medical support device 102 stores the received communication message in the communication message storage unit 101.

In a case in which the reply button 124 is operated in the patient message list 109 and the staff message list 119, a message transmission screen (not illustrated) for a reply is transmitted to the client terminals 13a to 13c. The message transmission screen for a return differs from the message transmission screen 64 illustrated in Fig. 10 in that it does not include the transmission destination designation field 65 and the patient designation field 66 and comprises the title input field 67 and the text input field 68. In a case in which a communication message is transmitted on the basis of the patient event information and the staff event information, the transmission destination designation field 65 and the patient designation field 66 are omitted in the message transmission screen for a reply since a target patient is known in advance and the transmission destination of the communication message to be replied is predetermined.

At the time that a title and text are input to the message transmission screen for a reply and the transmission button is operated, a communication message including a transmission destination ID, a transmission source ID, the transmission date and time, a target patient ID, and a patient event ID or a staff event ID, in addition to the information input through the message transmission screen, is transmitted from the client terminals 13a to 13c to the team medical support device 102, similarly to the transmission of the message by the new button 123. The team medical support device 102 stores the received communication message in the communication message storage unit 101.

In a case in which there is no communication message related to the selected patient event information and the selected staff event information, the patient message list 109 and the staff message list 119 are not displayed in the patient-related message display region 103b and the staff-related message display region 104b. Therefore, a small screen (not illustrated) in which the new button 123 for newly transmitting a communication message is provided is displayed in the patient-related message display region 103b and the staff-related message display region 104b.

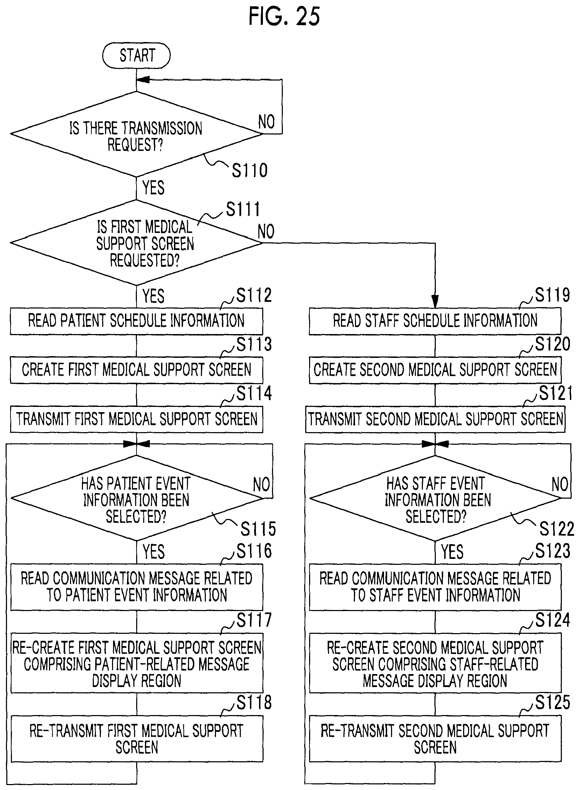

Next, the operation of the above-mentioned structure will be described with reference to a flowchart illustrated in Fig. 25. The team medical support device 102 waits for a medical support screen transmission request from the client terminals 13a to 13c (S110). At the time that a transmission request from the client terminals 13a to 13c is received (YES in S110), the screen creation unit 51 specifies the type of medical support screen corresponding to the transmission request (S111).

In a case in which the transmission of the first medical support screen 103 is requested in the transmission request (YES in S111), the screen creation unit 51 reads the patient schedule information of a patient designated by the transmission request from the patient schedule information storage unit 16 (S112). The screen creation unit 51 creates the first medical support screen 103 having only the patient schedule display region 103a on the basis of the read patient schedule information (S113). At the time that the creation of the first medical support screen 103 is completed, the transmission unit 52 transmits the first medical support screen 103 to the client terminals 13a to 13c which have transmitted to the transmission request (S114).

The client terminals 13a to 13c which have received the first medical support screen 103 display the first medical support screen 103 on the displays. The patient schedule table 107 in which the patient schedule information read from the patient schedule information storage unit 16 is arranged in the form of a table is displayed in the patient schedule display region 103a of the first medical support screen 103. In the patient schedule table 107, the date and time is allocated in chronological order along the horizontal axis and the vertical axis and patient event information is displayed in each of a plurality of cells 108 which correspond to the date and time and are arranged in a matrix.

The team medical support device 102 waits for the selection of patient event information from the patient schedule table 107 in the client terminals 13a to 13c (S115). In a condition of receiving patient event selection information from the client terminals 13a to 13c (YES in S115), the screen creation unit 51 reads a communication message related to the patient event ID which is designated by the patient event selection information from the communication message storage unit 101 (S116) and re-creates the first medical support screen 103 comprising the patient-related message display region 103b on the basis of the read communication message (S117). The transmission unit 52 re-transmits the re-created first medical support screen 103 to the client terminals 13a to 13c (S118).

The patient message list 109 in which the communication messages related to the selected patient event information are summarized is displayed in the patient-related message display region 103b of the first medical support screen 103. Therefore, the patient schedule information and the patient-related messages are displayed in parallel on the first medical support screen 103.

In a case in which the transmission of the second medical support screen 104 is requested in the transmission request (NO in S111), the screen creation unit 51 reads the staff schedule information of a medical staff member designated by the transmission request from the staff schedule information storage unit 17 (S119). The screen creation unit 51 creates the second medical support screen 104 comprising only the staff schedule display region 104a on the basis of the read staff schedule information (S120). At the time that the creation of the second medical support screen 104 is completed, the transmission unit 52 transmits the second medical support screen 104 to the client terminals 13a to 13c which have transmitted to the transmission request (S121).

The client terminals 13a to 13c which have received the second medical support screen 104 display the second medical support screen 104 on the displays. The staff schedule table 117 in which the staff schedule information read from the staff schedule information storage unit 17 is arranged in the form of a table is displayed in the staff schedule display region 104a of the second medical support screen 104. In the staff schedule table 117, the date and time is allocated in chronological order along the horizontal axis and the vertical axis and staff event information is displayed in each of a plurality of cells 118 which correspond to the date and time and are arranged in a matrix.

The team medical support device 102 waits for the selection of staff event information from the staff schedule table 117 in the client terminals 13a to 13c (S122). In a condition of receiving staff event selection information from the client terminals 13a to 13c (YES in S122), the screen creation unit 51 reads a communication message related to the staff event ID which is designated by the staff event selection information from the communication message storage unit 101 (S123) and re-creates the second medical support screen 104 comprising the staff-related message display region 104b on the basis of the read communication message (S124). The transmission unit 52 re-transmits the re-created second medical support screen 104 to the client terminals 13a to 13c (S125).

The staff message list 119 in which the communication messages related to the selected staff event information are summarized is displayed in the staff-related message display region 104b of the second medical support screen 104. Therefore, the staff schedule information and the staff-related messages are displayed in parallel on the second medical support screen 104.

As described above, the team medical support device 102 according to this embodiment creates the first medical support screen 103 and the second medical support screen 104 comprising the patient-related message display region 103b and the staff-related message display region 104b in which the communication messages related to the patient event information and the staff event information are summarized and displayed, respectively, and transmits the first medical support screen 103 and the second medical support screen 104 to the client terminals 13a to 13c which have transmitted the transmission request. It is possible to collectively check communication messages related to a medical procedure for a patient indicated by patient event information and the work of the medical staff indicated by staff event information through the first medical support screen 103 and the second medical support screen 104. Therefore, it is possible to accurately and rapidly ascertain the content of the communication message and the medical staff smoothly exchanges and shares information in a team medical treatment.

In the second embodiment, one patient event information item and one staff event information item are selected from the patient schedule table 107 and the staff schedule table 117, respectively. However, in a case in which a plurality of event information items are selected, a plurality of message lists in which communication messages related to each of the selected event information items are summarized may be displayed. In addition, the text of the communication message is displayed in the message display field of the message list. However, a title may also be displayed. The communication messages are displayed in the order of the transmission date and time. However, the display order may be appropriately changed. For example, the communication messages are displayed in the order of the degree of importance, the degree of urgency, the medical staff , or unconfirmed messages. The patient message list 109 and the staff message list 119 are displayed so as to be superimposed on the patient schedule table 107 and the staff schedule table 117, respectively. However, the patient schedule table 107 and the staff schedule table 117 may be displayed in parallel in the horizontal direction.

### [Third Embodiment]

In the first embodiment, all of the communication messages related to a patient are displayed in the patient-related message display region 20b of the first medical support screen 20. However, the job type of the medical staff who participates in a team medical treatment is not limited to a doctor, a nurse, an X-ray technician, and a pharmacist, but is various. Therefore, it is necessary to protect detailed information related to a medical examination in terms of the protection of personal information or medical ethics, according to the job type of the medical staff. In this embodiment, the communication messages which are displayed in the patient-related message display region 20b of the first medical support screen 20 are protected according to the access authority of the medical staff of the medical staff such that the medical staff members smoothly exchange and share information in the team medical treatment.

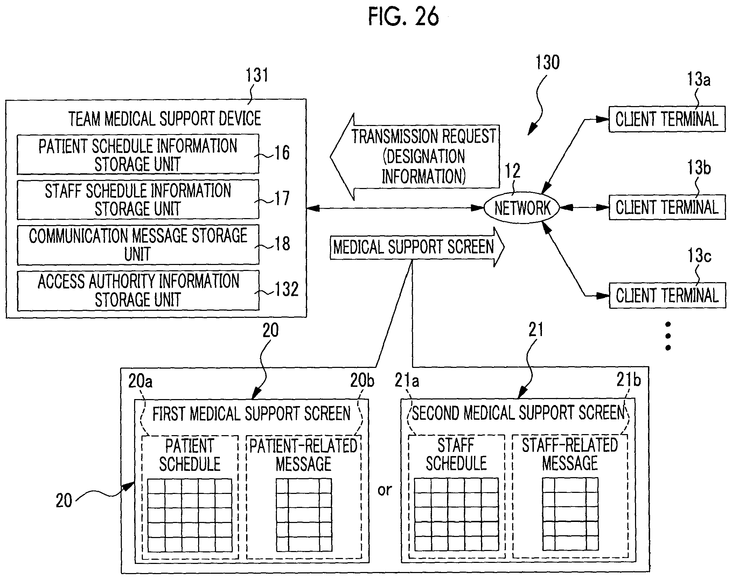

A team medical support device 131 of a team medical support system 130 illustrated in Fig. 26 differs from the team medical support device 11 according to the first embodiment in that it further includes an access authority information storage unit 132 that stores the access authority information of the medical staff for the patient-related message display region20b. For example, the type of communication message which is not allowed to be access is registered for each medical staff member and each job type of the medical staff in the access authority information storage unit 132. Examples of the type of communication message in which the access authority information is set include a communication message which is transmitted between medical staff members of a specify job type, such as a communication message which is transmitted from a doctor to another doctor, and a communication message which is transmitted between specific medical staff members, such as a communication message which is transmitted from a doctor D1 to a nurse N1. In addition, access authority may be set according to the degree of urgency of the communication message.

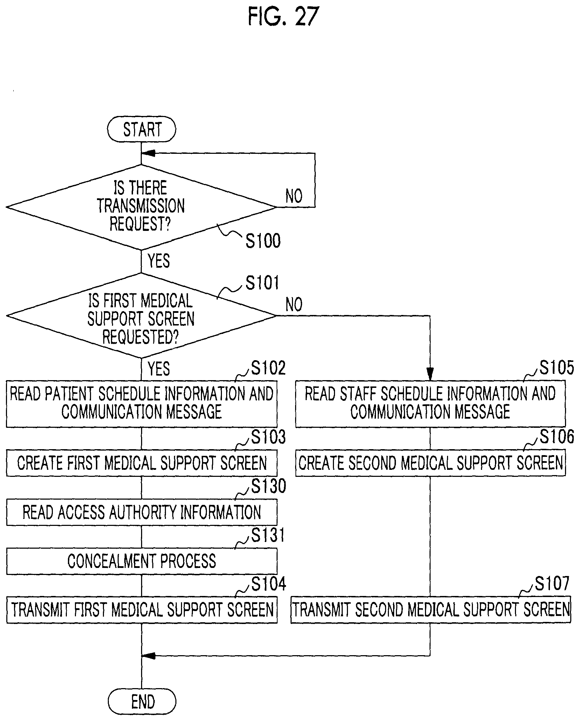

As illustrated in Fig. 27, a screen creation unit 51 of the team medical support device 131 receives a transmission request from the client terminals 13a to 13c (YES in S100). At the time that the transmission of a first medical support screen 20 is requested (YES in S101), the screen creation unit 51 reads the patient schedule information and communication message of a patient designated by the transmission request (S102) and creates the first medical support screen 20 on the basis of the read patient schedule information and communication message (S103).

The screen creation unit 51 reads the access authority information of a medical staff member who has transmitted the transmission request from the access authority information storage unit 132 (S130). In a case in which there is a communication message which is not allowed to be accessed by the access authority information among the communication messages displayed in the patient-related message display region 20b, the screen creation unit 51 performs a concealment process of concealing the communication message which is not allowed to be accessed from the patient-related message display region 20b (S131). After the communication message concealment process is completed, the transmission unit 52 transmits the first medical support screen 20 to the client terminals 13a to 13c which have transmitted the transmission request (S104).

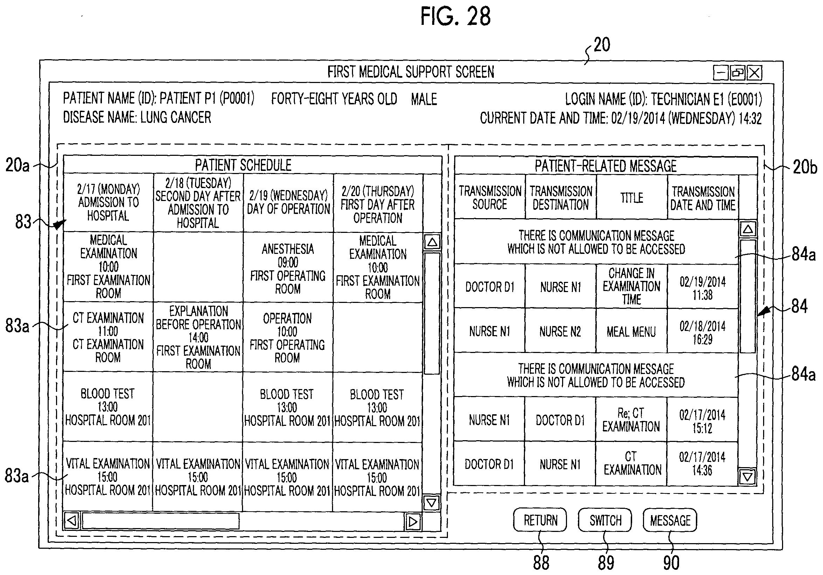

Fig. 28 illustrates a first medical support screen 103 of a patient P1 of which the transmission has been requested by a technician E1 who is an X-ray technician. In a patient-related message list 84 of a patient-related message display region 20b, a concealment process which prevents a communication message from being displayed is performed for a communication message display field 84a which is not allowed to be accessed by the technician E1. In addition, in the concealment process, a message "there is a communication message that is not allowed to be accessed" is displayed in the communication message display field 84a subjected to the concealment process.

As described above, in this embodiment, in a case in which the communication messages displayed in the patient-related message display region include a communication message which is not allowed to be accessed by a medical staff member to which the first medical support screen is transmitted, the team medical support device 131 performs the concealment process of concealing the communication message which is not allowed to be accessed. Therefore, it is possible to protect the personal information of patients or information to be protected in terms of medical ethics from the medical staff who does not have access authority. In addition, since information indicating that there is a communication message which is not allowed to be accessed is displayed in the display field of the communication message subjected to the concealment process, a medical staff member can recognize that there is a communication message which is not allowed to be accessed, participate in a team medical treatment, and smoothly cooperate with other medical staff members. Therefore, the medical staff members smoothly exchange and share information in the team medical treatment.

### [Fourth Embodiment]

In the first embodiment, the team medical support device 11 receives the communication messages transmitted from the client terminals 13a to 13c and stores the communication messages in the communication message storage unit 18. In a case in which a medical support screen transmission request is received from the client terminals 13a to 13c, the team medical support device 11 displays the communication messages on the first medical support screen 20 and the second medical support screen 21 and transmits the first medical support screen 20 and the second medical support screen 21 to the client terminals 13a to 13c. Therefore, at the time that the transmitted medical support screens are not received, a medical staff member is not capable of checking new communication messages transmitted from other medical staff members. As a result, the medical staff members do not smoothly exchange and share information. This embodiment provides a structure which enables medical staff members to rapidly recognize the presence of a new communication message and to smoothly exchange and share information in a team medical treatment.

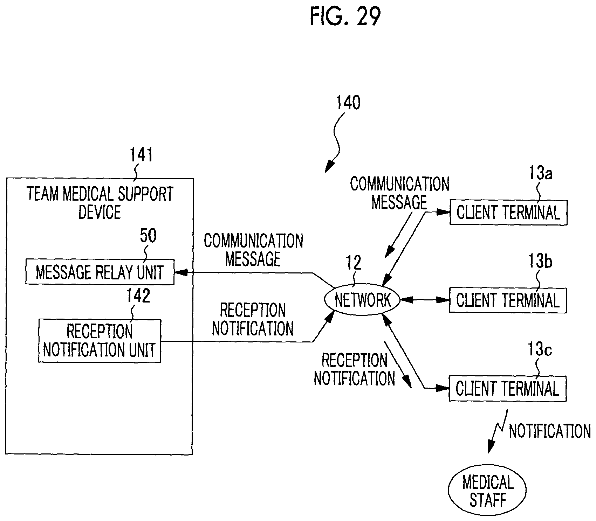

A team medical support device 141 of a team medical support system 140 illustrated in Fig. 29 differs from the team medical support device 11 according to the first embodiment in that it further includes a reception notification unit 142. The CPU 36 implements the functions of the reception notification unit 142 in cooperation with, for example, the memory 37, similarly to the message relay unit 50.

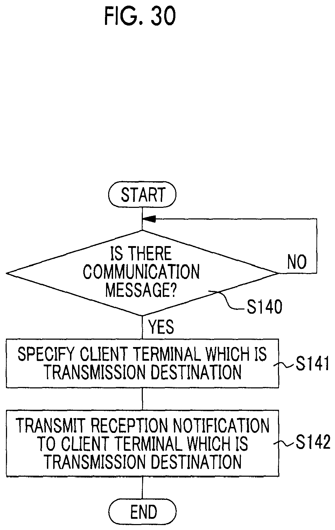

As illustrated in Fig. 30, the message relay unit 50 of the team medical support device 141 waits for the transmission of a communication message from the client terminals 13a to 13c (S140). For example, at the time that the message relay unit 50 receives a communication message from the client terminal 13a (YES in S130), the reception notification unit 142 specifies a client terminal which is the transmission destination of the received communication message (S141). The client terminal which is the transmission destination is specified by searching for a medical staff member having the same staff ID as the transmission destination ID of the received communication message from the staff information storage unit 25 and acquiring the terminal ID of the client terminal used by the searched medical staff member from the staff information storage unit 25. For example, in a case in which the client terminal which is the transmission destination is the client terminal 13c, the reception notification unit 142 transmits a notification indicating the reception of the communication message to the client terminal 13c (S142).

The client terminal 13c notifies the medical staff member of the reception of the communication message, using, for example, a sound, light, vibration, or the display of a screen. In a condition of receiving the communication message reception notification, the medical staff can request the team medical support device 141 to transmit a medical support screen, check the medical support screen, and check the content of the communication message.

As described above, in this embodiment, in a case in which a communication message is received from a client terminal, the team medical support device 141 transmits a reception notification to a client terminal which is the transmission destination of the received communication message. Therefore, a medical staff member who has received the communication message can rapidly recognize the presence of a communication message, without checking the medical support screen. As a result, the medical staff members smoothly exchange and share information in a team medical treatment.

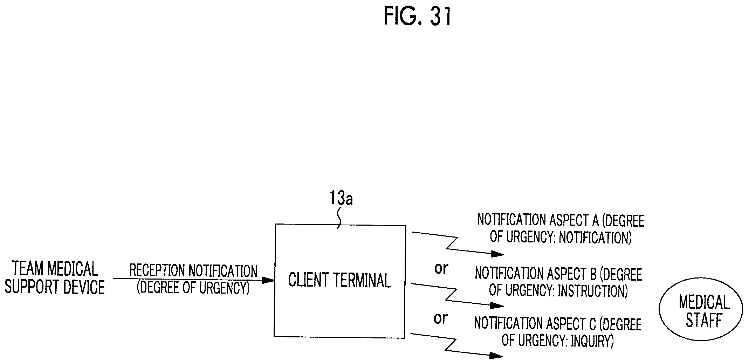

At the time that the reception of a communication message is notified to a medical staff member together with the degree of urgency of the communication message, the medical staff member can perform an operation corresponding to the degree of urgency. Therefore, medical staff members smoothly exchange and share information. As means for notifying the degree of urgency of the communication message, as illustrated in Fig. 31, the team medical support device 141 transmits a reception notification to which information about the degree of urgency is added to the client terminals 13a to 13c. In a condition of receiving the reception notification, the client terminal changes the aspect of the notification to the medical staff, depending on the degree of urgency added to the reception notification.

For example, in the case of "notification" having the highest degree of urgency of a communication message, a notification aspect A which is likely to draw the attention of the medical staff may be used. In the case of "inquiry" having a low degree of urgency, a notif ication aspect C which has a smaller effect of drawing attention of the medical staff than the notification aspect A may be used. In the case of "instruction" having the medium degree of urgency, a notification aspect B having an intermediate attention drawing function between the notification aspect A and the notification aspect C may be used. In a case in which a sound is used for notification, for example, different volumes or different types of sound may be used in the notification aspects A, B, and C. In a case in which light is used for notification, for example, different amounts of light or different light emission patterns may be used in the notification aspects A, B, and C. In addition, in a case in which vibration is used for notification, different vibration intensities or different vibration patterns may be used in the notification aspects A, B, and C.

### [Fifth Embodiment]

In the first embodiment, a text data communication message is transmitted from the client terminals 13a to 13c to the team medical support device 11, using the message transmission screen 64 illustrated in Fig. 10. However, in a busy medical practice, in some cases, it is difficult to make time to input a communication message to the message transmission screen 64 and to transmit the communication message. In the medical practice, since the number of times the communication message is transmitted is reduced, the exchange or sharing of information between medical staff members is sluggish in a team medical treatment. As a method for simply transmitting a communication message in a short time, a method is considered which inputs the communication message as audio data using a standard sound input function of the client terminals 13a to 13c. However, in some cases, it is necessary to listen to a communication message, which is audio data, to the end in order to ascertain the content of the communication message, which results in an increase in the time required to check the communication message. This embodiment enables the medical staff to rapidly ascertain the content of a communication message which is input as audio data such that medical staff members smoothly exchange and share information in a team medical treatment.

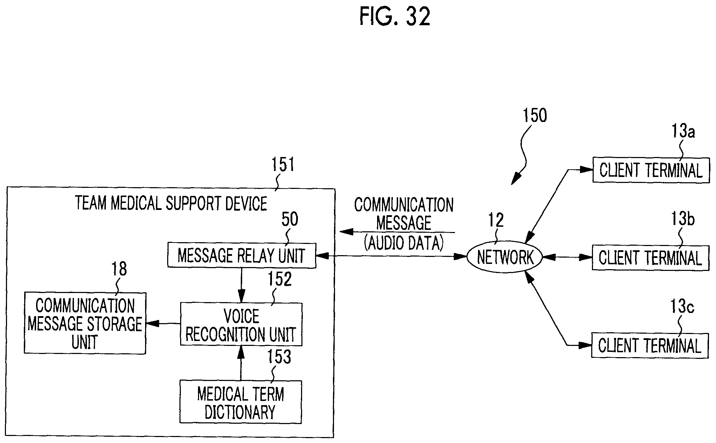

A team medical support device 151 of a team medical support system 150 illustrated in Fig. 32 differs from the team medical support device 11 according to the first embodiment in that it further includes a voice recognition unit 152 and a medical term dictionary 153. The voice recognition unit 152 analyzes audio data and creates text data. The medical term dictionary 153 is dictionary data in which medical terms are registered. In a condition of analyzing audio data and converting the audio data into text data, the voice recognition unit 152 creates text data in which the medical terms are appropriately used, with reference to the medical term dictionary 153.

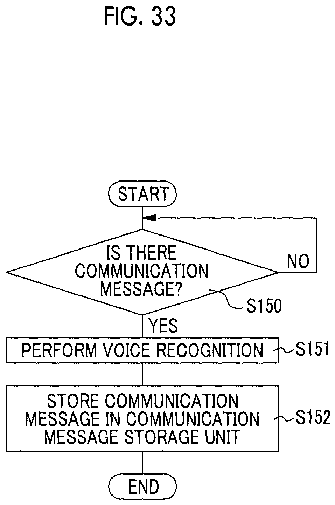

As illustrated in Fig. 33, the message relay unit 50 of the team medical support device 151 waits for the transmission of communication messages from the client terminals 13a to 13c (S150). The client terminals 13a to 13c input a communication message, which is audio data, using the standard sound input function, and transmit the communication message to the team medical support device 151. At the time that the message relay unit 50 receives the communication message which is audio data (YES in S150), the voice recognition unit 152 converts the audio data into text data with reference to the medical term dictionary 153 (S151). The voice recognition unit 152 stores the communication message which has been converted into the text data in the communication message storage unit 18 (S152). In a case in which a medical support screen transmission request is received from the client terminals 13a to 13c, the team medical support device 151 reads the communication message which has been converted into the text data from the communication message storage unit 18, creates a medical support screen, and transmits the created medical support screen to the client terminals 13a to 13c which have transmitted the transmission request.

As described above, the team medical support device 151 according to this embodiment converts a communication message which is input as audio data into text data, creates a medical support screen using the converted text data, and transmits the medical support screen. Therefore, the medical staff can rapidly ascertain the content of the communication message, without listening to the audio data to the end. As a result, medical staff members smoothly exchange and share information in a team medical treatment.

In this embodiment, in a condition of recognizing audio data, the voice recognition unit 152 specifies medical terms in the audio data with reference to the medical term dictionary 153. At the time that a medical support screen is created using the specification result of the medical terms, the medical terms in the communication message may be displayed so as to be highlighted. As an example of the highlighted display, the color or font of the medical terms may be different from that of data other than the medical terms or the medical terms may be underlined. In this case, it is easy to know the position where medical terms are used in the communication message and it is possible to rapidly ascertain the content of the communication message. Therefore, medical staff members smoothly exchange and share information in a team medical treatment.

### [Sixth Embodiment]

There is a communication message for instructing a treatment or an examination for a patient. At the time that the communication message for instructing a treatment or an examination for a patient is used only for information exchange or information sharing, it is not useful to smoothly perform a team medical treatment. In this embodiment, the communication message for instructing a treatment or an examination for a patient is effectively used.

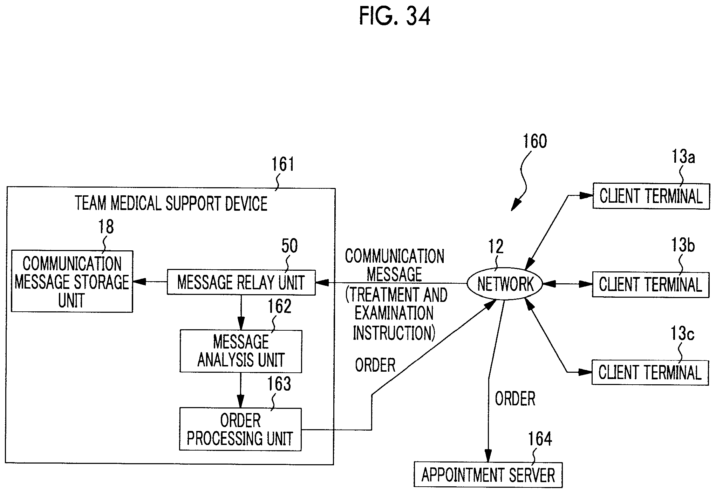

A team medical support device 161 of a team medical support system 160 illustrated in Fig. 34 differs from the team medical support device 11 according to the first embodiment in that it further includes a message analysis unit 162 and an order processing unit 163. An appointment server 164 for an appointment for a treatment or an examination is connected to the team medical support device 161 through a network 12. The message analysis unit 162 analyzes the title or text of communication messages and specifies a communication message for instructing a treatment or an examination for a patient. The order processing unit 163 transmits information about an appointment for a treatment or an examination to the appointment server 164 on the basis of the content of the communication message for instructing a treatment or an examination for a patient.

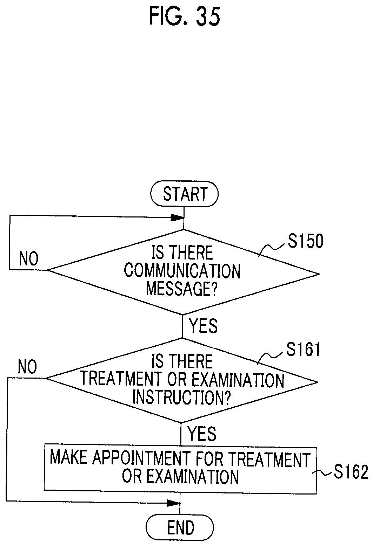

As illustrated in Fig. 35, the message relay unit 50 of the team medical support device 161 waits for the transmission of communication messages from the client terminals 13a to 13c (S160). The client terminals 13a to 13c transmit a communication message for instructing a treatment or an examination for a patient or a communication message for smooth information exchange or information sharing to the team medical support device 161. At the time that the message relay unit 50 receives the communication messages (YES in S160), the message analysis unit 162 analyzes the received communication messages and specifies a communication message for instructing a treatment or an examination for a patient (S161). In a case in which the message analysis unit 162 specifies the communication message for instructing a treatment or an examination for a patient (YES in S161), the order processing unit 163 transmits information about an appointment for a treatment or an examination to the appointment server 164 on the basis of the content of the specified communication message (S162).

As described above, the team medical support device 161 according to this embodiment makes an appointment for a treatment or an examination on the basis of the communication message for instructing a treatment or an examination for a patient. Therefore, the communication message for instructing a treatment or an examination for a patient can be effectively used for purposes other than smooth information exchange or information sharing.

In each of the above-described embodiments, at the time that a communication message is transmitted, the staff ID of a medical staff member who is a transmission destination is designated. However, for example, the patient ID of a patient may be designated and a communication message may be transmitted to all of the medical staff members related to a medical examination for the patient. In addition, at the time that the transmission of the second medical support screen 21 is requested, a medical staff member is designated. However, the transmission request may be made using the staff ID of a medical staff member who logs in the team medical support device 11. In this case, the designation of the medical staff member can be omitted.

The switching button 89 is operated to switch between the first medical support screen 20 and the second medical support screen 21. However, in a case in which a portable terminal, such as a smart phone or a tablet terminal, is used as the client terminals 13a to 13c, the first medical support screen 20 and the second medical support screen 21 may be switched in operative association with the rotation of the portable terminal at the time that the direction of the screen is changed. In this case, the portable terminal can be rotated on the basis of the detection result of a standard sensor of the portable terminal.

In each of the above-described embodiments, the team medical support device is installed in the medical institutions. However, the team medical support device may be installed in, for example, data centers or local medical centers. In this case, the team medical support device is connected to the client terminals 13a to 13c in the medical institution through, for example, the Internet, a dedicated communication line, or a virtual private network (VPN). In each of the above-described embodiments, for example, the patient schedule information storage unit 16, the staff schedule information storage unit 17, the communication message storage unit 18, the patient information storage unit 24, and the staff information storage unit 25 are table-type databases. However, databases in which each patient, each medical staff member, and each communication message are managed as files may be used.

## Claims

1. A team medical support device comprising:
a patient schedule information storage unit that stores patient schedule information of a plurality of patients in which patient event information indicating a medical procedure that is scheduled to be performed for the patient is registered;
a staff schedule information storage unit that stores staff schedule information of a plurality of staff members in which staff event information indicating work that is scheduled to be performed by the medical staff member is registered;
a communication message storage unit that stores a plurality of communication messages which are exchanged between client terminals used by the plurality of medical staff members;
a receiving unit that receives a medical support screen transmission request including designation information for designating one patient or one medical staff member among the plurality of patients or the plurality of medical staff members;
a screen creation unit that creates two types of medical support screens, that is, a first medical support screen and a second medical support screen, on the basis of the patient schedule information, the staff schedule information, and the communication messages; and
a transmission unit that transmits the first medical support screen or the second medical support screen which is created on the basis of the transmission request to the client terminals,
wherein a patient schedule display region in which the patient schedule information of the patient designated by the designation information is displayed, and a patient-related message display region in which a communication message related to the patient designated by the designation information or the patient event information of the patient is displayed, are displayed in parallel on the first medical support screen, and
a staff schedule display region in which the staff schedule information of the medical staff member designated by the designation information is displayed, and a staff-related message display region in which a communication message related to the medical staff member designated by the designation information or the staff event information of the medical staff member is displayed, are displayed in parallel on the second medical support screen.

2. The team medical support device according to claim 1,
wherein a transmission date and time of the communication message is displayed in the patient-related message display region and the staff-related message display region.

3. The team medical support device according to claim 1,
wherein a transmission source and a transmission destination of the communication message are displayed in at least one of the patient-related message display region or the staff-related message display region.

4. The team medical support device according to claim 1,
wherein a title of the communication message or at least a portion of text of the communication message is displayed in at least one of the patient-related message display region or the staff-related message display region.

5. The team medical support device according to claim 1,
wherein the screen creation unit displays the communication messages in the patient-related message display region in an order of a transmission date and time and sets a display order of the communication messages in the staff-related message display region according to a degree of urgency of the communication message.

6. The team medical support device according to claim 1, further comprising:
an access authority information storage unit that stores access authority information of the medical staff member for the patient-related message display region,
wherein, in a condition of creating the first medical support screen, the screen creation unit checks the access authority information of the medical staff member, who is a request source requesting the transmission of the first medical support screen, and
in a case in which the communication messages displayed in the patient-related message display region include the communication message that is not allowed to be accessed by the medical staff member, the screen creation unit performs a concealment process of concealing the communication message that is not allowed to be accessed.

7. The team medical support device according to claim 6,
wherein, in the concealment process, information indicating that the communication message that is not allowed to be accessed by the medical staff member is present is displayed.

8. The team medical support device according to claim 1, further comprising:
a message relay unit that receives the communication message transmitted from the client terminal and relays the communication message; and
a reception notification unit that transmits a notification indicating the reception of the communication message to the client terminal used by the medical staff member who is a transmission destination of the received communication message.

9. The team medical support device according to claim 1, further comprising:
a voice recognition unit that, in a case in which audio data is received as the communication message from the client terminal, converts the audio data into text data and stores the text data in the communication message storage unit.

10. The team medical support device according to claim 9,
wherein the voice recognition unit converts medical terms in the audio data into text data on the basis of a medical term dictionary that stores the medical terms.

11. The team medical support device according to claim 1, further comprising:
a message analysis unit that analyzes the content of the communication message received from the client terminal; and
an order processing unit that, in a case in which the content of the communication message instructs a treatment or an examination for a patient, orders the treatment or the examination on the basis of the content of the communication message.

12. A method for controlling a team medical support device, comprising:
a receiving step of receiving a medical support screen transmission request including designation information for designating one patient or one medical staff member among a plurality of patients or a plurality of medical staff members;
an information reading step of accessing a patient schedule information storage unit that stores patient schedule information of a plurality of patients in which patient event information indicating a medical procedure that is scheduled to be performed for the patient is registered, a staff schedule information storage unit that stores staff schedule information of a plurality of staff members in which staff event information indicating a work that is scheduled to be performed by the medical staff member is registered, and a communication message storage unit that stores a plurality of communication messages which are exchanged between client terminals used by the plurality of medical staff members, and reading the patient schedule information, the staff schedule information, and the communication messages;
a screen creation step of creating two types of medical support screens, that is, a first medical support screen and a second medical support screen, on the basis of the patient schedule information, the staff schedule information, and the communication messages; and
a transmission step of transmitting the first medical support screen or the second medical support screen which is created on the basis of the transmission request to the client terminals,
wherein a patient schedule display region in which the patient schedule information of the patient designated by the designation information is displayed, and a patient-related message display region in which a communication message related to the patient designated by the designation information or the patient event information of the patient is displayed, are displayed in parallel on the first medical support screen, and
a staff schedule display region in which the staff schedule information of the medical staff member designated by the designation information is displayed, and a staff-related message display region in which a communication message related to the medical staff member designated by the designation information or the staff-related information of the medical staff member is displayed, are displayed in parallel on the second medical support screen.

13. A team medical support program that causes a computer to perform:
a receiving step of receiving a medical support screen transmission request including designation information for designating one patient or one medical staff member among a plurality of patients or a plurality of medical staff members;
an information reading step of accessing a patient schedule information storage unit that stores patient schedule information of a plurality of patients in which patient event information indicating a medical procedure that is scheduled to be performed for the patient is registered, a staff schedule information storage unit that stores staff schedule information of a plurality of staff members in which staff event information indicating a work that is scheduled to be performed by the medical staff member is registered, and a communication message storage unit that stores a plurality of communication messages which are exchanged between client terminals used by the plurality of medical staff members, and reading the patient schedule information, the staff schedule information, and the communication messages;
a screen creation step of creating two types of medical support screens, that is, a first medical support screen and a second medical support screen, on the basis of the patient schedule information, the staff schedule information, and the communication messages; and
a transmission step of transmitting the first medical support screen or the second medical support screen which is created on the basis of the transmission request to the client terminals,
wherein a patient schedule display region in which the patient schedule information of the patient designated by the designation information is displayed, and a patient-related message display region in which a communication message related to the patient designated by the designation information or the patient event information of the patient is displayed, are displayed in parallel on the first medical support screen, and
a staff schedule display region in which the staff schedule information of the medical staff member designated by the designation information is displayed, and a staff-related message display region in which a communication message related to the medical staff member designated by the designation information or the staff event information of the medical staff member is displayed, are displayed in parallel on the second medical support screen.

14. A team medical support system comprising:
client terminals that are used by a plurality of medical staff members; and
a team medical support device that creates a medical support screen on the basis of a transmission request from the client terminals and transmits the medical support screen to the client terminals,
wherein the team medical support device comprises
a patient schedule information storage unit that stores patient schedule information of a plurality of patients in which patient event information indicating a medical procedure that is scheduled to be performed for the patient is registered,
a staff schedule information storage unit that stores staff schedule information of a plurality of staff members in which staff event information indicating a work that is scheduled to be performed by the medical staff member is registered,
a communication message storage unit that stores a plurality of communication messages which are exchanged between the client terminals used by the plurality of medical staff members,
a receiving unit that receives a medical support screen transmission request including designation information for designating one patient or one medical staff member among the plurality of patients or the plurality of medical staff members,
a screen creation unit that creates two types of medical support screens, that is, a first medical support screen and a second medical support screen, on the basis of the patient schedule information, the staff schedule information, and the communication messages, and
a transmission unit that transmits the first medical support screen or the second medical support screen which is created on the basis of the transmission request to the client terminals,
a patient schedule display region in which the patient schedule information of the patient designated by the designation information is displayed, and a patient-related message display region in which a communication message related to the patient designated by the designation information or the patient event information of the patient is displayed, are displayed in parallel on the first medical support screen, and
a staff schedule display region in which the staff schedule information of the medical staff member designated by the designation information is displayed, and a staff-related message display region in which a communication message related to the medical staff member designated by the designation information or the staff event information of the medical staff member is displayed, are displayed in parallel on the second medical support screen.
